# EUROPÄISCHE PATENTSCHRIFT

(11) **EP 0 609 328 B1**
(45) Veröffentlichungstag und Bekanntmachung des Hinweises auf die Patenterteilung: **28.04.1999**
(21) Anmeldenummer: 92922034.1
(22) Anmeldetag: 21.10.1992
(51) Int. Cl.: C07D 487/04, A61K 31/50

(54) **PYRROLO-PYRIDAZINE MIT MAGEN- UND DARMSCHUTZWIRKUNGEN**
PYRROLO-PYRIDAZINES WITH GASTRO-INTESTINAL PROTECTIVE ACTION
PYRROLO-PYRIDAZINE A EFFETS PROTECTEURS DU TUBE GASTRO-INTESTINAL

(30) Priorität: 25.10.1991 CH 3119/91; 25.10.1991 CH 3120/91; 25.10.1991 CH 3123/91
(43) Veröffentlichungstag der Anmeldung: 10.08.1994
(73) Patentinhaber: BYK GULDEN LOMBERG CHEMISCHE FABRIK GMBH, D-78403 Konstanz (DE)
(72) Erfinder: GRUNDLER, Gerhard, D-7750 Konstanz (DE); RAINER, Georg, D-7750 Konstanz (DE); SCHAEFER, Hartmann, D-7750 Konstanz 18 (DE); SENN-BILFINGER, Jörg, D-7750 Konstanz (DE); SIMON, Wolfgang-Alexander, D-7750 Konstanz (DE); RIEDEL, Richard, D-7967 Bad Waldsee-Reute (DE); POSTIUS, Stefan, D-7750 Konstanz (DE); STURM, Ernst, D-7750 Konstanz 18 (DE); HUBER, Reinhard, D-7753 Allensbach 2 (DE); DAVID, Michael, D-7750 Konstanz 19 (DE)
(86) Internationale Anmeldenummer: EP9202418
(87) Internationale Veröffentlichungsnummer: WO9308190

(56) Entgegenhaltungen:
- WO-A-92/06979
- US-A- 4 988 695

## Beschreibung

### Anwendungsgebiet der Erfindung

Die Erfindung betrifft neue Verbindungen, Verfahren zu ihrer Herstellung und ihre Anwendung als Wirkstoffe in Arzneimitteln.

### Bekannter technischer Hintergrund

Im J. Heterocyclic Chem. 10, 551 (1973) sind auf bestimmte Weise substituierte Pyrrolopyridazine beschrieben. - Im U.S. Patent 4,988,695 werden Pyrrolo- und Dihydropyrrolocinnoline und ihre Verwendung als Magensäuresekretionshemmer beschrieben.

### Beschreibung der Erfindung

Gegenstand der Erfindung sind neue Verbindungen der Formel I (siehe beiliegendes Formelblatt),
worin
- R1: 1-4C-Alkyl oder durch R5 substituiertes 1-3C-Alkylen,
- R2: 1-4C-Alkyl,
- R3: Wasserstoff, Halogen, CHO (Formyl), Hydroxymethyl, Nitro, Amino oder den Substituenten -CH₂O-COR₇,
- R4: Halogen oder den Substituenten -A-B-R6,
- R5: Furyl, Thienyl, Tetrahydrofuryl, Phenyl oder durch einen oder zwei gleiche oder verschiedene Substituenten aus der Gruppe Halogen, 1-4C-Alkyl und 1-4C-Alkoxy substituiertes Phenyl,
- R6: Wasserstoff, Thienyl, Furyl, Phenyl oder durch einen oder zwei gleiche oder verschiedene Substituenten aus der Gruppe Halogen, 1-4C-Alkyl, 1-4C-Alkoxy, Nitro, -NH-CO-NH₂ (Ureido), Amino, 1-4C-Alkylcarbonylamino und 1-4C-Alkoxycarbonylamino substituiertes Phenyl,
- R7: 1-4C-Alkyl, 1-4C-Alkoxy-1-4C-alkyl, 1-4C-Alkoxycarbonyl-1-4C-alkyl oder Carboxy-1-4C-alkyl,
- A: 0 (Sauerstoff) oder NH,
- B: einen Bindungsstrich, -CH₂- (Methylen) oder -CH₂CH₂- (1,2-Ethylen) und
- n: die Zahl 0 oder 1 darstellt,
und die Salze dieser Verbindungen.
1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.
1-3C-Alkylen steht für Trimethylen, Ethylen und insbesondere Methylen.
Halogen im Sinne der vorliegenden Erfindung ist Brom, Chlor und Fluor.
1-4C-Alkoxyreste enthalten neben dem Sauerstoffatom einen der vorstehend genannten 1-4C-Alkylreste. Bevorzugt ist der Methoxyrest.
1-4C-Alkylcarbonylreste in der 1-4C-Alkylcarbonylaminogruppe enthalten neben der Carbonylgruppe einen der vorstehend genannten 1-4C-Alkylreste. Bevorzugt ist der Acetylrest.
1-4C-Alkoxycarbonylreste in der 1-4C-Alkoxycarbonylaminogruppe enthalten neben der Carbonylgruppe einen der vorstehend genannten 1-4C-Alkoxyreste. Bevorzugt sind der Methoxycarbonyl- und der Ethoxycarbonylrest.
1-4C-Alkoxy-1-4C-alkyl steht für die vorstehend genannten 1-4C-Alkylreste, an die einer der vorstehend genannten 1-4C-Alkoxyreste gebunden ist. Bevorzugt ist der Methoxymethylrest.
1-4C-Alkoxycarbonyl-1-4C-alkyl steht für die vorstehend genannten 1-4C-Alkylreste, an die ein 1-4C-Alkoxycarbonylrest (Carbonyl mit einem der vorstehend genannten 1-4C-Alkoxyreste) gebunden ist. Bevorzugt ist der Methoxycarbonylmethyl- und der Methoxycarbonyl ethyl rest.
Carboxy-1-4C-alkyl steht für die vorstehend genannten 1-4C-Alkylreste, an die ein Carboxylrest (-COOH) gebunden ist. Bevorzugt sind der Carboxymethyl- und der Carboxyethylrest.

Als Salze kommen für Verbindungen der Formel I bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 2-Hydroxy-3-naphthoat oder Mesilat.

Eine Ausgestaltung (Ausgestaltung a) der Erfindung sind Verbindungen der Formel I, worin R3 Wasserstoff, R6 Wasserstoff, B einen Bindungsstrich und n die Zahl 0 darstellt, und R1, R2, R4, R5 und A die eingangs angegebenen Bedeutungen haben, und ihre Salze. Diese Verbindungen sind neue Zwischenprodukte zur Herstellung der pharmakologisch wirksamen Endprodukte.

Eine weitere Ausgestaltung (Ausgestaltung b) der Erfindung sind Verbindungen der Formel I, worin R1, R2, R3, R4, R5, R6, A, B und n die eingangs angegebenen Bedeutungen haben, mit Ausnahme der Bedeutung Wasserstoff für R3 und R6, mit Ausnahme der Bedeutung Halogen für R4 und mit Ausnahme der Bedeutung Bindungsstrich für B, und ihre Salze.

Hervorzuhebende Verbindungen der Ausgestaltung b sind solche der Formel I, worin
- R1: 1-4C-Alkyl oder durch R5 substituiertes 1-3C-Alkylen,
- R2: 1-4C-Alkyl,
- R3: Hydroxymethyl, Nitro, Amino oder den Substituenten -CH₂O-COR7,
- R4: den Substituenten -A-B-R6,
- R5: Furyl, Thienyl, Tetrahydrofuryl, Phenyl oder durch einen oder zwei gleiche oder verschiedene Substituenten aus der Gruppe Halogen, 1-4C-Alkyl und 1-4C-Alkoxy substituiertes Phenyl,
- R6: Thienyl, Furyl, Phenyl oder durch einen oder zwei gleiche oder verschiedene Substituenten aus der Gruppe Halogen, 1-4C-Alkyl, 1-4C-Alkoxy, Nitro, -NH-CO-NH₂ (Ureido), Amino, 1-4C-Alkylcarbonylamino und 1-4C-Alkoxycarbonylamino substituiertes Phenyl,
- R7: 1-4C-Alkyl, 1-4C-Alkoxy-1-4C-alkyl, 1-4C-Alkoxycarbonyl -1-4C-alkyl oder Carboxy-1-4C-alkyl,
- A: 0 (Sauerstoff) oder NH,
- B: -CH₂- (Methylen) oder -CH₂CH₂- (1,2-Ethylen) und
- n: die Zahl 0 oder 1 darstellt,
und die Salze dieser Verbindungen.

Besonders hervorzuhebende Verbindungen der Ausgestaltung b sind solche der Formel I, worin
- R1: Isobutyl oder durch R5 substituiertes Methylen,
- R2: 1-4C-Alkyl,
- R3: Hydroxymethyl, Nitro, Amino oder den Substituenten -CH₂O-COR7,
- R4: den Substituenten -A-B-R6,
- R5: Furyl, Thienyl, Phenyl oder durch einen oder zwei gleiche oder verschiedene Substituenten aus der Gruppe Halogen, 1-4C-Alkyl und 1-4C-Alkoxy substituiertes Phenyl,
- R6: Thienyl, Phenyl oder durch einen oder zwei gleiche oder verschiedene Substituenten aus der Gruppe Halogen, 1-4C-Alkyl, 1-4C-Alkoxy, -NH-CO-NH₂ (Ureido), Amino, 1-4C-Alkylcarbonylamino und 1-4C-Alkoxycarbonylamino substituiertes Phenyl,
- R7: 1-4C-Alkyl, 1-4C-Alkoxy-1-4C-alkyl, 1-4C-Alkoxycarbonyl-1-4C-alkyl oder Carboxy-1-4C-alkyl,
- A: 0 (Sauerstoff) oder NH,
- B: -CH₂- (Methylen) und
- n: die Zahl 0 oder 1 darstellt,
und die Salze dieser Verbindungen.

Bevorzugte Verbindungen der Ausgestaltung b sind solche der Formel I, worin
- R1: durch R5 substituiertes Methylen,
- R2: 1-4C-Alkyl,
- R3: Hydroxymethyl, Amino oder den Substituenten -CH₂O-COR7,
- R4: den Substituenten -A-B-R6,
- R5: Phenyl oder durch einen Substituenten aus der Gruppe Chlor, Fluor und 1-4C-Alkoxy substituiertes Phenyl,
- R6: Phenyl oder durch einen Substituenten aus der Gruppe Chlor und Fluor substituiertes Phenyl,
- R7: Methyl, Methoxymethyl, Methoxycarbonylmethyl, Methoxycarbonylethyl, Carboxymethyl oder Carboxyethyl,
- A: 0 (Sauerstoff),
- B: -CH₂- (Methylen) und
- n: die Zahl 0 darstellt,
und die Salze dieser Verbindungen.

Besonders bevorzugte Verbindungen der Ausgestaltung b sind solche der Formel I, worin
- R1: durch R5 substituiertes Methylen,
- R2: 1-4C-Alkyl,
- R3: Hydroxymethyl, Amino oder den Substituenten -CH₂O-COR7,
- R4: den Substituenten -A-B-R6,
- R5: Phenyl oder durch Methoxy substituiertes Phenyl,
- R6: Phenyl oder durch Fluor substituiertes Phenyl,
- R7: Methyl, Methoxymethyl oder Carboxyethyl,
- A: 0 (Sauerstoff),
- B: -CH₂- (Methylen) und
- n: die Zahl 0 darstellt,
und die Salze dieser Verbindungen.

Als beispielhafte erfindungsgemäße Verbindungen seien die in der folgenden Tabelle 1 aufgeführten Verbindungen der Formel I (siehe Formelblatt) mit ihren Substituentenbedeutungen und die Salze dieser Verbindungen genannt (Ph steht dabei für Phenyl, die Zahlen geben die Positionen der Substituenten am Phenylring an):

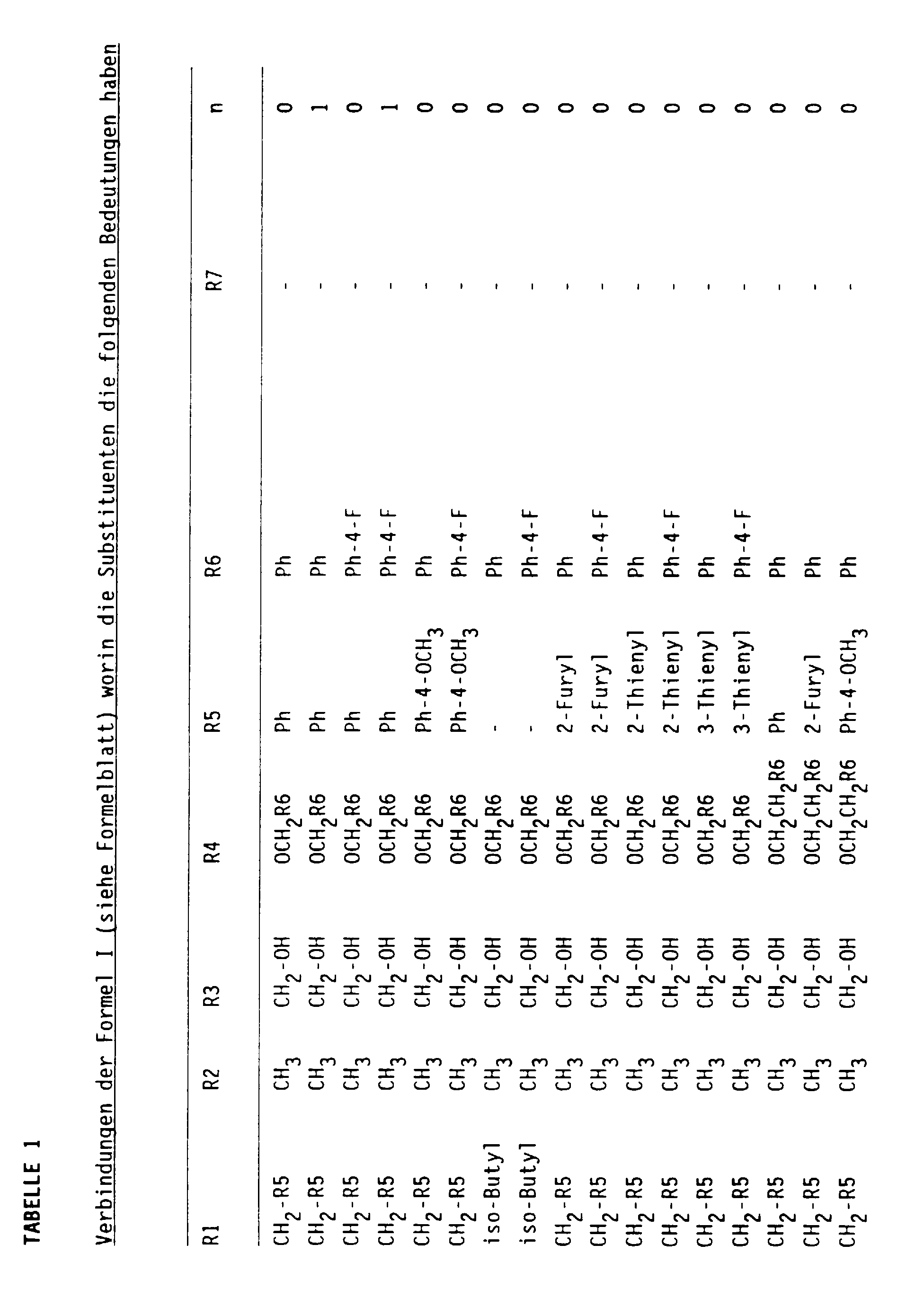

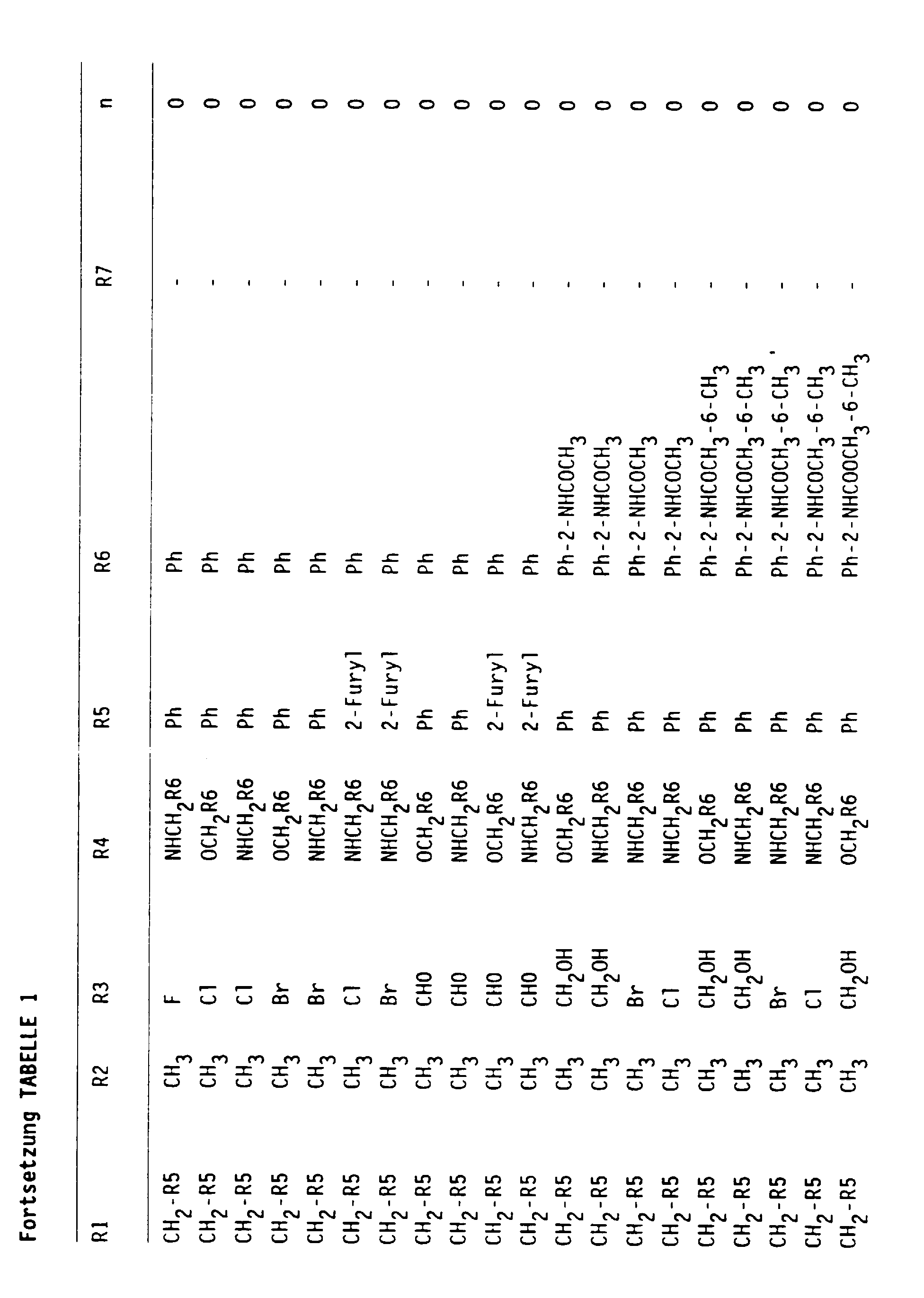

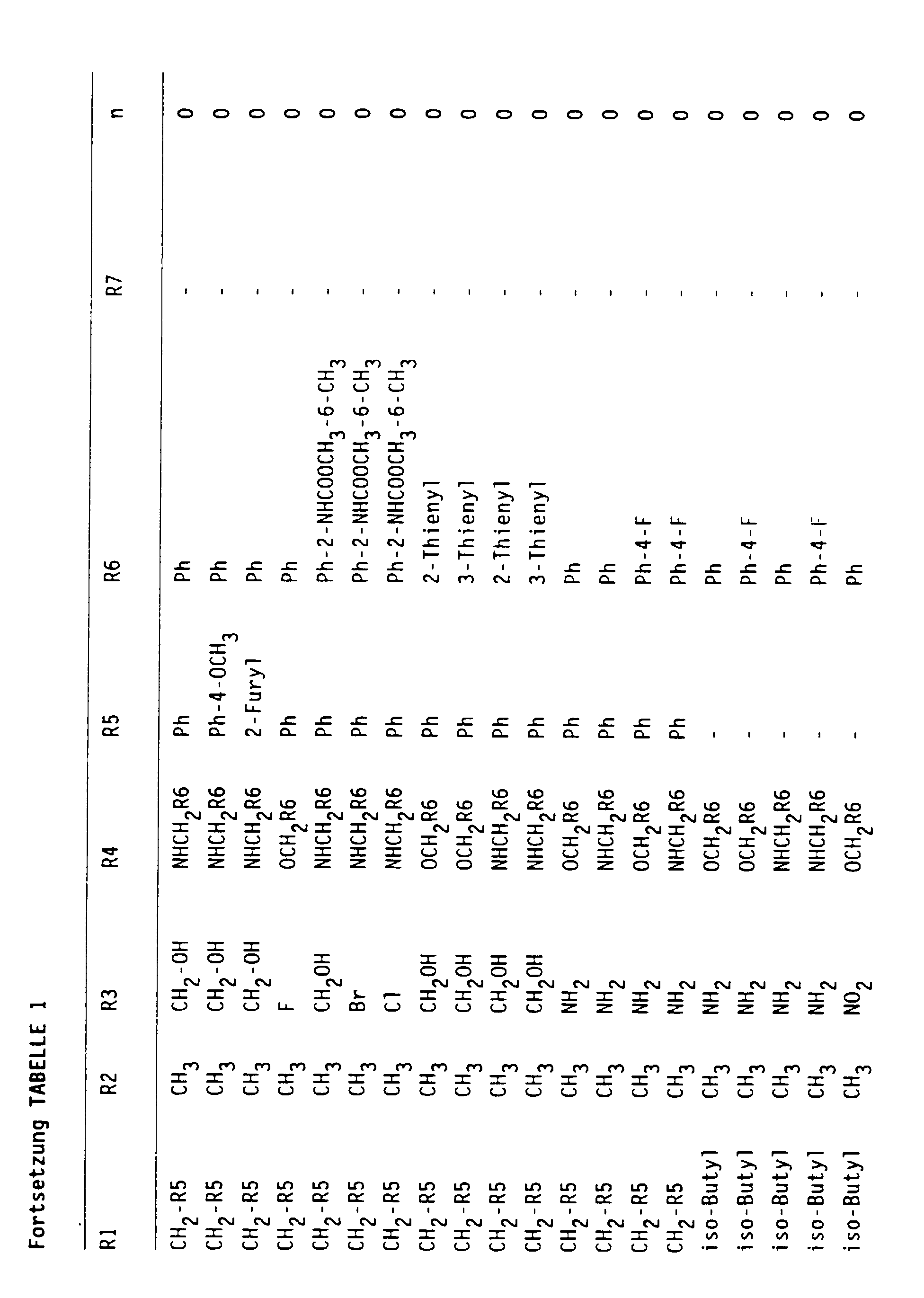

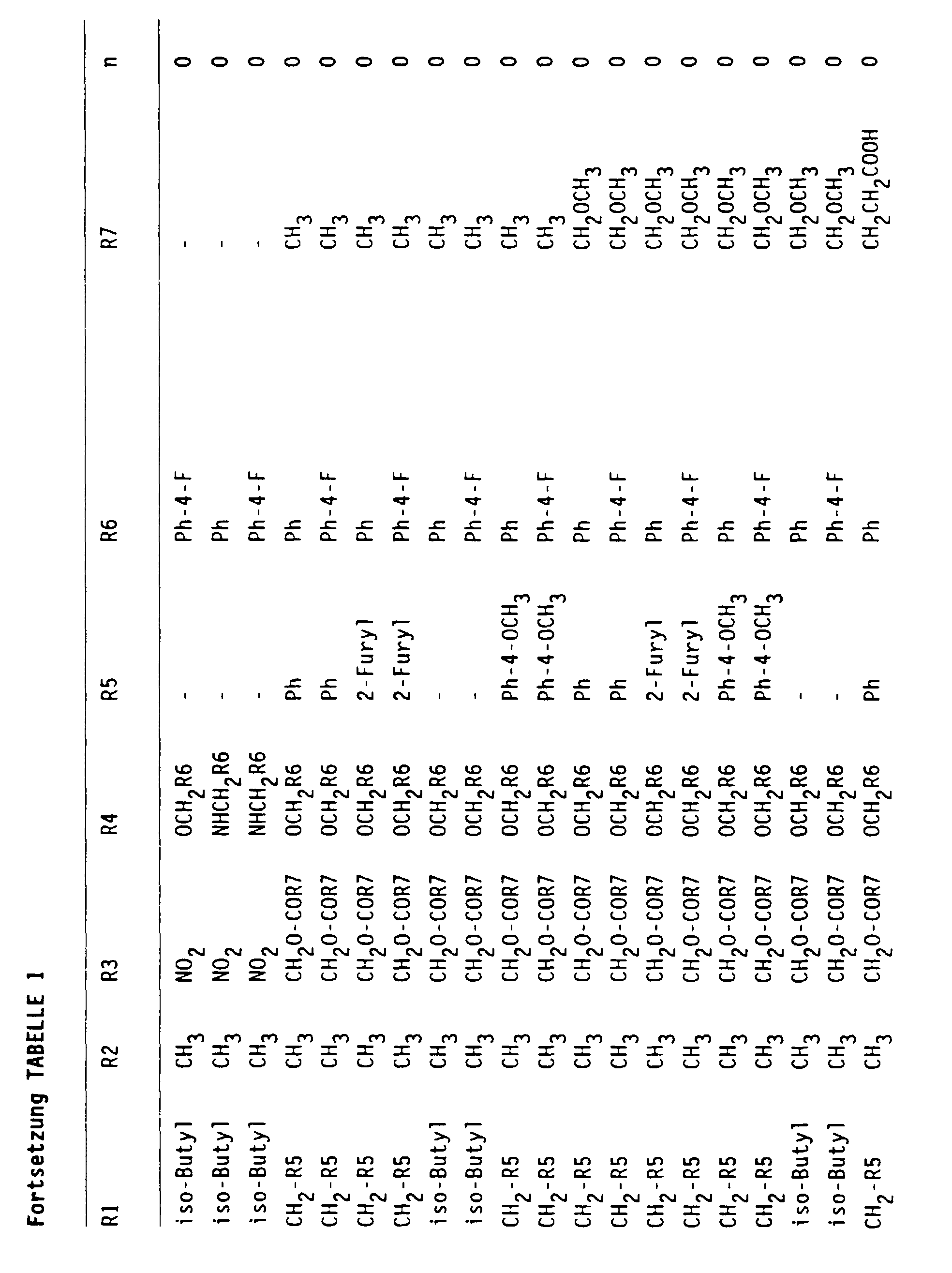

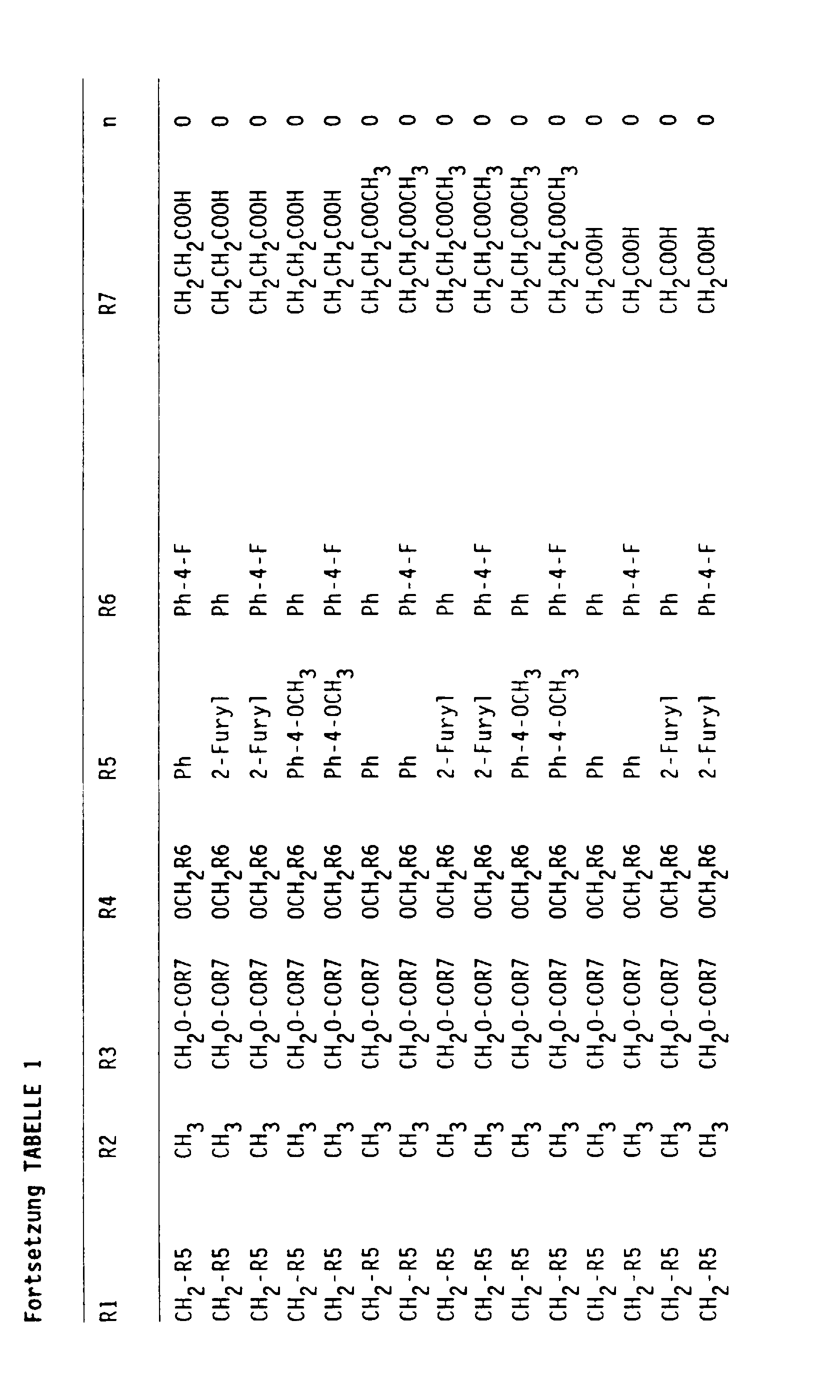

Weiterer Gegenstand der Erfindung ist ein Verfahren zur Herstellung der erfindungsgemäßen Verbindungen und ihrer Salze. Das Verfahren ist dadurch gekennzeichnet, daß man
a) zur Herstellung von Verbindungen I, in denen R3 und R6 Wasserstoff, R4 den Substituenten -A-B-R6, A Sauerstoff, B einen Bindungsstrich und n die Zahl 0 bedeutet, Verbindungen der Formel II (siehe Formelblatt), worin R1 und R2 die oben angegebenen Bedeutungen haben und Hal ein Halogenatom, vorzugsweise ein Chloratom darstellt, reduktiv debenzyliert und enthalogeniert, oder daß man
b) zur Herstellung von Verbindungen I, in denen R3 Wasserstoff, R4 Halogen und n die Zahl 0 bedeutet, die gemäß a) erhaltenen Verbindungen der Formel III (siehe Formelblatt), worin R1 und R2 die oben angegebenen Bedeutungen haben, mit geeigneten Halogenierungsmitteln umsetzt, oder daß man
c) zur Herstellung von Verbindungen I, in denen R3 Wasserstoff, R4 den Substituenten -A-B-R6 und n die Zahl 0 bedeutet, die gemäß b) erhaltenen Verbindungen der Formel IV (siehe Formelblatt), worin R4 Halogen bedeutet, mit Verbindungen der Formel H-A-B-R6 (= Verbindungen der Formel V), worin A, B und R6 die oben angegebenen Bedeutungen haben, oder ihren Salzen mit Basen, umsetzt, oder daß man
d) zur Herstellung von Verbindungen I, in denen R3 Halogen und n die Zahl 0 bedeutet, Verbindungen der Formel IV, worin R1, R2 und R4 die oben angegebenen Bedeutungen haben, mit geeigneten Halogenierungsmitteln umsetzt, oder daß man
e) zur Herstellung von Verbindungen I, in jenen R3 CHO (Formyl) und n die Zahl 0 bedeutet, Verbindungen der Formel I, worin R3 Haloge- bedeutet, in 3-Position intermediär metalliert und anschließend mit Dimethylformamid oder Ameisensäureester umsetzt, oder daß man
f) zur Herstellung von Verbindungen I, in denen R3 Hydroxymethyl und n die Zahl 0 bedeutet, Verbindungen der Formel I, worin R3 CHO (Formyl) bedeutet, reduziert, oder daß man
g) zur Herstellung von Verbindungen I, in denen n die Zahl 1 bedeutet, Verbindungen der Formel I, worin n die Zahl 0 bedeutet, oxydiert, oder daß man
h) zur Herstellung von Verbindungen I, in denen R4 den Rest -A-B-R6 bedeutet, wobei R6 durch 1-4C-Alkoxycarbonylamino oder 1-4C-Alkylcarbonylamino substituiertes Phenyl bedeutet, Verbindungen der Formel I, worin R4 den Rest -A-B-R6 bedeutet, wobei R6 durch Amino substituiertes Phenyl bedeutet, mit Halogenameisensäure-1-4C-alkylester bzw. mit 1-4C-Carbonsäurehalogenid umsetzt, oder daß man
i) zur Herstellung von Verbindungen I, in denen R3 Nitro und R4 Halogen bedeutet, Verbindungen der Formel I, worin R3 Wasserstoff und R4 Halogen bedeutet, nitriert, oder daß man
j) zur Herstellung von Verbindungen I, in denen R3 Nitro und R4 den Substituenten -A-B-R6 bedeutet, die gemäß i) erhaltenen Verbindungen der Formel I, worin R3 Nitro und R4 Halogen bedeutet, mit Verbindungen der Formel H-A-B-R6 (= Verbindungen der Formel V), worin A, B, und R6 die oben angegebenen Bedeutungen haben, oder ihren Salzen mit Basen, umsetzt, oder daß man
k) zur Herstellung von Verbindungen I, in denen R3 Amino und R4 den Substituenten -A-B-R6 bedeutet, die gemäß j) erhaltenen Verbindungen der Formel I, worin R3 Nitro und R4 den Substituenten -A-B-R6 bedeutet, reduziert, oder daß man
l) zur Herstellung von Verbindungen I, in denen R3 den Substituenten -CH₂O-COR7 und R7 1-4C-Alkyl, 1-4C-Alkoxy-1-4C-alkyl, 1-4C-Alkoxycarbonyl-1-4C-alkyl oder Carboxy-1-4C-alkyl bedeutet, Verbindungen der Formel I, worin R3 Hydroxymethyl bedeutet, mit Verbindungen der Formel R7-CO-Z (= Verbindungen der Formel VI) umsetzt, worin R7 die vorstehenden Bedeutungen hat und Z OH (Hydroxy) oder eine geeignete Abgangsgruppe darstellt,
und daß man gewünschtenfalls anschließend die nach a), b), c), d), e), f), g), h), i), j), k) oder l) erhaltenen Verbindungen I in ihre Salze überführt, oder daß man gewünschtenfalls anschließend aus erhaltenen Salzen der Verbindungen I die Verbindungen I freisetzt.

Die reduktive Debenzylierung und Enthalogenierung gemäß Verfahrensvariante a) erfolgt in einer für den Fachmann geläufigen Weise, z.B. mit Wasserstoff in Gegenwart von Palladium als Katalysator, in Methanol bei Raumtemperatur.

Die Halogenierung der Verbindungen der Formel III erfolgt in Gegenwart geeigneter oder vorzugsweise ohne Lösungsmittel. Als geeignete Lösungsmittel kommen nur wasserfreie Solventien, wie z.B. offenkettige oder cyclische Ether (Diethylether, Diethylenglycoldimethylether, Dioxan oder Tetrahydrofuran), oder (aromatische) Kohlenwasserstoffe, wie z.B. Cyclohexan, Benzol oder Toluol infrage, die sich gegenüber dem Halogenierungsmittel inert verhalten.

Als Halogenierungsmittel kommen alle bekannten halogenabspaltenden Reagenzien infrage, insbesondere solche Verbindungen, die für einen Einsatz im technischen Maßstab geeignet sind. Beispielsweise seien Phosphortrichlorid, Phospnortribromid, Thionylchlorid, Thionylbromid, Phosphoroxytrichlorid oder Phosphoroxytribromid genannt.

Die Halogenierung wird (je nach Art des Halogenierungsmittels und des gegebenenfalls eingesetzten Lösungsmittels) bei Temperaturen zwischen 0° und 150°C, insbesondere bei der Siedetemperatur des verwendeten Lösungs- bzw. Halogenierungsmittels durchgeführt.

Die Umsetzung der Verbindungen IV mit den Verbindungen der Formel V gemäß Verfahrensvariante c) erfolgt (je nach Art der Verbindung V) in einem wasserfreien, inerten Lösungsmittel oder ohne weiteren Lösungsmittelzusatz unter Verwendung eines Überschusses an Verbindung V als Solvens. Als wasserfreie, inerte Lösungsmittel kommen insbesondere aprotische, polare Lösungsmittel, wie z.B. Dimethylsulfoxid, Tetrahydrofuran, Dioxan oder Dimethylformamid infrage.

Je nach Art der Verbindungen V erfordert ihre Umsetzung mit den Verbindungen IV die Gegenwart einer Hilfsbase bzw. (wenn in Verbindung V A die Bedeutung NH hat) einen Überschuß an Verbindung V. Als Hilfsbasen kommen beispielsweise organische Amine (wie Triethylamin oder Diisopropylamin), Alkalicarbonate (wie Natriumcarbonat oder Kaliumcarbonat) oder Alkalihydroxide (wie Natriumhydroxid oder Kaliumhydroxid), bevorzugt jedoch solche Verbindungen infrage, die in der Lage sind, die Verbindungen V glatt zu deprotonieren. Hier seien insbesondere Metallhydride (z.B. Natriumhydrid) oder Alkalimetalle (z.B. Natrium) erwähnt, die entweder vor der Umsetzung mit IV zur Deprotonierung der Verbindung V eingesetzt oder dem Reaktionsgemisch aus IV und V zugegeben werden. In einer bevorzugten Verfahrensvariante kann die Deprotonierung auch durch ein Alkalialkoholat, wie beispielsweise Kalium-tert.-butylat, in Gegenwart eines Kronenethers, wie beispielsweise [18]Krone-6 erfolgen.

Die Reaktionstemperatur liegt - je nach Reaktivität der Verbindung V bzw. ihrer Salze mit Basen - zwischen 0° und 150°C, wobei bei höherer Reaktivität Temperaturen zwischen 0° und 50° ausreichen, bei geringerer Reaktivität jedoch höhere Temperaturen erforderlich sind, wobei die Siedetemperatur des verwendeten Lösungsmittels bzw. des als Lösungsmittel verwendeten Überschusses an Verbindung V bevorzugt ist.

Die Halogenierung gemäß Verfahrensvariante d) erfolgt auf eine dem Fachmann geläufige Weise, beispielsweise durch direkte Umsetzung mit dem Halogen in geeigneten Lösungsmitteln, beispielsweise in Dichlormethan, bei Temperaturen von vorzugsweise um oder unter 0°C, oder durch Umsetzung mit geeigneten Halogenierungsmitteln, wie beispielsweise N-Brom- oder N-Chlorsuccinimid, in inerten Lösungsmitteln, wie etwa Dichlormethan oder Dimethylformamid.

Die intermediäre Metallierung gemäß Verfahrensvariante e) kann beispielsweise in Form einer Grignard-Reaktion oder unter Verwendung von Butyllithium als Reagens erfolgen. Die Metallierung erfolgt unter Reaktionsbedingungen und in Lösungsmitteln, wie sie dem Fachmann für die Durchführung von Reaktionen mit metallorganischen Verbindungen geläufig sind. Die anschließende Umsetzung mit Dimethylformamid oder Ameisensäureester erfolgt ebenfalls in an sich bekannter Weise.

Die Reduktion der Formylgruppe zur Hydroxymethylgruppe gemäß Verfahrensvariante f) erfolgt ebenfalls auf eine dem Fachmann vertraute Weise, beispielsweise durch Umsetzung mit Natriumborhydrid in einem geeigneten Lösungsmittel, wie z.B. Ethanol.

Die N-Oxidation gemäß Verfahrensvariante g) erfolgt in an sich bekannter Weise unter Verwendung üblicher Oxidationsmittel, wobei die N-Oxidation bevorzugt unter Verwendung von m-Chlorperoxibenzoesäure in Dichlormethan bei Raumtemperatur durchgeführt wird.

Die Überführung der Aminogruppe in die Alkoxycarbonylamino- bzw. Alkylcarbonylaminogruppe gemäß Verfahrensvariante h) erfolgt in einer für die Urethan- bzw. Amidherstellung an sich bekannten Weise, bevorzugt durch Umsetzung der Aminoverbindungen mit entsprechenden Chlorameisensäureestern bzw. mit entsprechenden Carbonsäurechloriden.

Die Nitrierung gemäß Verfahrensvariante i) erfolgt auf eine dem Fachmann vertraute Weise mit Nitriersäure (Salpetersäure/Schwefelsäure) unter den üblichen Reaktionsbedingungen.

Die Umsetzung gemäß Verfahrensvariante j) erfolgt in analoger Weise wie für Verfahrensvariante c) beschrieben.

Die Reduktion gemäß Verfahrensvariante k) wird unter möglichst schonenden Bedingungen durchgeführt, beispielsweise unter Verwendung von Natriumdithionit als Reduktionsmittel und unter Reaktionsbedingungen, wie diese bei der Anwendung dieses Reduktionsmittels üblich sind.

Die Umsetzung der Verbindungen I mit R3 = Hydroxymethyl mit den Verbindungen VI gemäß Verfahrensvariante 1) erfolgt in an sich bekannter Weise, wie sie dem Fachmann aufgrund seines Fachwissens über Veresterungsreaktionen bekannt ist. Die Veresterung erfolgt in inerten Lösungsmitteln, wie beispielsweise Dioxan oder Tetrahydrofuran, und je nach Art der Gruppe Z entweder in Gegenwart eines wasserabspaltenden bzw. Wasser chemisch bindenden Mittels, wie beispielsweise Dicyclohexylcarbodiimid (wenn Z = OH), oder in Gegenwart einer Hilfsbase (z.B. Triethylamin), wenn Z eine Abgangsgruppe, beispielsweise ein Halogenatom (insbesondere Chlor) darstellt. Wenn R7 einen Carboxy-1-4C-alkylrest darstellt, wird als Abgangsgruppe Z bevorzugt ein Alkoxycarbonyloxyrest (gemischtes Anhydrid), insbesondere der Isobutoxycarbonyloxyrest, ohne weiteren Zusatz eines wasserabspaltenden Mittels eingesetzt.

Die Isolierung und Reinigung der nach den Verfahrensvarianten a) bis 1) erhaltenen erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Verbindungen in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Verbindungen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Verbindungen der Formel II können nach dem im beigefügten Formelblatt angegebenen allgemeinen Syntheseschema hergestellt werden, beispielsweise so, wie dies in den nachfolgenden Beispielen exemplarisch ausgeführt ist.

Die im Syntheseschema als Ausgangsverbindungen aufgeführten Pyrrole sind bekannt [siehe z.B. R.G. Jones, J. Am. Chem. Soc. 77, 4069 (1955); ibid. 78, 159 (1956)] oder sie können auf an sich bekannte Weise aus bekannten Verbindungen hergestellt werden.

Die folgenden Beispiele erläutern die Erfindung näher ohne sie einzuschränken. Die in den Beispielen namentlich aufgeführten Verbindungen der allgemeinen Formel I sowie die Salze dieser Verbindungen sind bevorzugter Gegenstand der Erfindung. Schmp.: bedeutet Schmelzpunkt, für Stunde(n) wird die Abkürzung h und für Minuten die Abkürzung Min. verwendet. Unter "Ether" wird Diethylether verstanden.

### Beispiele:

### End- und Zwischenprodukte

### 1a. 1-Benzyl-7-(4-fluorbenzyloxy)-3-hydroxymethyl-2-methyl-pyrrolo-[2,3-d]pyridazin

Eine Lösung von 780 mg (2,08 mMol) 1-Benzyl-7-(4-fluorbenzyloxy)-3-formyl -2-methyl-pyrrolo[2,3-d]pyridazin in 50 ml Ethanol wird mit 80 mg (2,1 mMol) Natriumborhydrid versetzt und 30 Min. bei Raumtemperatur gerührt. Nach Zugabe von 100 ml Wasser wird die Mischung am Rotationsverdampfer auf die Hälfte des Volumens eingeengt. Der Niederschlag wird abfiltriert, mit 50 ml Wasser gewaschen und im Hochvakuum über Kaliumhydroxid getrocknet. 670 mg (85%) der Titelverbindung werden isoliert. Schmp.: 122-124°C.

### 1b. 1-Benzyl-7-benzyloxy-3-hydroxymethyl-2-methyl-pyrrolo[2,3-d]pyridazin

1,3 g (3,6 mmol) 1-Benzyl-7-benzyloxy-3-formyl-2-methyl-pyrrolo[2,3-d]-pyridazin und 140 mg (3,7 mmol) Natriumborhydrid werden in 125 ml Ethanol, wie für Beispiel 1a. beschrieben, umgesetzt. Ausbeute: 92%, Schmp.: 130-132°C.

### 1c. 1-Benzyl-7-benzylamino-3-hydroxymethyl-2-methyl-pyrrolo[2,3-d]pyridazin

0,2 g (0,56 mmol) 1-Benzyl-7-benzylamino-3-formyl-2-methyl-pyrrolo[2,3-d]-pyridazin und 23 mg (0,62 mmol) Natriumborhydrid werden in 20 ml Methanol, wie für Beispiel la. beschrieben, umgesetzt. Ausbeute: 80%, Schmp.: 238-240°C.

### 2a. 1-Benzyl-7-(4-fluorbenzyloxy)-3-formyl-2-methyl-pyrrolo[2,3-d] pyridazin

Eine Lösung von 810 mg (1,9 mMol) 1-Benzyl-3-brom-7-(4-fluorbenzyloxy)-2-methyl-pyrrolo[2,3-d]pyridazin in 30 ml wasserfreiem Tetrahydrofuran wird unter Argon auf -70°C gekühlt und mit 1,65 ml (2,7 mMol) einer 15%igen Lösung von n-Butyllithium in Hexan versetzt. Anschließend wird noch 30 Min. bei -70°C gerührt. Nach Zugabe von 215 µl (2,7 mMol) wasserfreiem Dimethylformamid wird die Temperatur noch weitere 30 Min. bei -70°C gehalten und anschließend langsam auf 20°C erhöht. Die Lösung wird dann mit 100 ml Wasser versetzt und mit 3 x 50 ml Essigester extrahiert. Die organischen Extrakte werden mit 50 ml Wasser gewaschen, über Magnesiumsulfat getrocknet und eingeengt. Der verbleibende Rückstand wird an Kieselgel (Fließmittel: Essigester) chromatographiert. Nach Einengen der Fraktionen mit Rf = 0,4 und Kristallisation aus Diisopropylether werden 150 mg (21%) der Titelverbindung isoliert. Schmp.: 136-141°C.

### 2b. 7-Benzyloxy-3-formyl-1-(4-methoxybenzyl)-2-methyl-pyrrolo[2,3-d] pyridazin

440 mg (1,0 mMol) 7-Benzyloxy-3-brom-1-(4-methoxybenzyl)-2-methyl-pyrrolo[2,3-d]pyridazin, 735 µl (1,2 mMol) 15%ige Lösung von n-Butyllithium in Hexan und 160 µl (1,2 mMol) Dimethylformamid werden in 25 ml Tetrahydrofuran, wie für Beispiel 2a. beschrieben, umgesetzt. Ausbeute: 36%, Schmp.: 139-145°C.

### 2c. 1-Benzyl-7-benzyloxy-3-formyl-2-methyl-pyrrolo[2,3-d]pyridazin

5,0 g (12,2 mmol) 1-Benzyl-7-benzyloxy-3-brom-2-methyl-pyrrolo[2,3-d]pyridazin, 9 ml (14,7 mmol) 15%ige Lösung von n-Butyllithium in Hexan und 2,4 ml (29,4 mmol) Dimethylformamid werden in 125 ml wasserfreiem Tetrahydrofuran, wie für Beispiel 2a. beschrieben, umgesetzt. Ausbeute: 32%, Schmp.: 147-149°C.

### 2d. 1-Benzyl-7-benzylamino-3-formyl-2-methyl-pyrrolo[2,3-d]pyridazin

0,58 g (1,4 mmol) 1-Benzyl-7-benzylamino-3-brom-2-methyl-pyrrolo[2,3-d]-pyridazin, 3,5 ml (5,7 mmol) 15%ige Lösung von n-Butyllithium in Hexan und 0,55 ml (7 mmol) Dimethylformamid werden in 40 ml wasserfreiem Tetrahydrofuran, wie für Beispiel 2a. beschrieben, umgesetzt. Ausbeute: 44%, Schmp.: 171-172°C.

### 3a. 1-Benzyl-3-brom-7-(4-fluorbenzyloxy)-2-methyl-pyrrolo[2,3-d]-pyridazin

Zu einer Lösung von 910 mg (2,6 mMol) 1-Benzyl-7-(4-fluorbenzyloxy)-2-methyl-pyrrolo[2,3-d]pyridazin in 15 ml wasserfreiem Dichlormethan wird bei 0°C eine Lösung von 180 µl (3,5 mMol) Brom in 7 ml wasserfreiem Dichlormethan während 30 Min. zugetropft. Die Lösung wird noch 1 h bei 0°C gehalten, anschließend mit 50 ml Eiswasser versetzt und extrahiert. Die organische Phase wird abgetrennt, mit 3 x 25 ml Wasser gewaschen, über Magnesiumsulfat getrocknet und eingeengt. Der Rückstand wird an Kieselgel (Fließmittel: Essigester) chromatographiert. Die Fraktionen mit Rf = 0,6 werden eingeengt und aus Diisopropylether/Cyclohexan kristallisiert. 880 mg (79%) der Titelverbindung werden isoliert. Schmp.: 128-130°C.

### 3b. 7-Benzyloxy-3-brom-1-(4-methoxybenzyl)-2-methyl-pyrrolo[2,3-d]pyridazin

1,8 g (5 mMol) 7-Benzyloxy-1-(4-methoxybenzyl)-2-methyl-pyrrolo[2,3-d]pyridazin und 282 µl (5,5 mMol) Brom werden in insgesamt 65 ml Dichlormethan, wie für Beispiel 3a. beschrieben, umgesetzt und gereinigt. Ausbeute: 78%, Schmp.: 149-152°C.

### 3c. 1-Benzyl-7-benzyloxy-3-brom-2-methyl-pyrrolo[2,3-d]pyridazin

42,3 g (0,128 mol) 1-Benzyl-7-benzyloxy-2-methyl-pyrrolo[2,3-d]pyridazin und 6,6 ml (0,128 mol) Brom werden in insgesamt 200 ml Dichlormethan, wie für Beispiel 3a. beschrieben, umgesetzt. Ausbeute: 85%, Schmp.: 122- 123°C.

### 3d. 1-Benzyl-3-brom-7-chlor-2-methyl-pyrrolo[2,3-d]pyridazin

8,0 g (31 mmol) 1-Benzyl-7-chlor-2-methyl-pyrrolo[2,3-d]pyridazin und 1,59 ml (31 mmol) Brom werden in insgesamt 150 ml Dichlormethan, wie für Beispiel 3a. beschrieben, umgesetzt. Ausbeute: 83%, Schmp.: 138-140°C.

### 4a. 1-Benzyl-7-(4-fluorbenzyloxy)-2-methyl-pyrrolo[2,3-d]pyridazin

Zu einer Lösung von 21,8 g (173 mMol) 4-Fluorbenzylalkohol in 150 ml wasserfreiem N-Methylpyrrolidon werden nacheinander 32,65 g (291 mMol) Kalium-tert.-butylat und 1,54 g (5,82 mMol) [18]Krone-6 (98%ig) gegeben. Diese Mischung wird 1 h bei Raumtemperatur gerührt. Anschließend wird eine Lösung von 15,0 g (58,2 mMol) 1-Benzyl-7-chlor-2-methyl-pyrrolo[2,3-d]pyridazin in 100 ml wasserfreiem N-Methylpyrrolidon während 30 Min. zugetropft. Es wird noch 15 Min. bei Raumtemperatur gerührt, danach mit 500 ml Eiswasser versetzt und mit 3 x 400 ml Essigester extrahiert. Die organischen Extrakte werden mit 3 x 300 ml Wasser gewaschen und anschließend über Magnesiumsulfat getrocknet. Nach Einengen und Kristallisation aus Diisopropylether werden 12,95 g (64%) der Titelverbindung isoliert. Schmp.: 127-132°C.

Auf analoge Weise werden hergestellt:

### 4b. 7-Benzyloxy-1-(4-methoxybenzyl)-2-methyl-pyrrolo[2,3-d]ridazin

5,63 g (52 mMol) Benzylalkohol, 90 ml N-Methylpyrrolidon, 9,75 g (87 mMol) Kalium-tert.-butylat, 0,46 g (1,74 mMol) [18]Krone-6 und 5,0 g (17 mMol) 7-Chlor-1-(4-methoxybenzyl)-2-methyl-pyrrolo[2,3-d]pyridazin werden 2 h bei Raumtemperatur umgesetzt. Reinigung: Kristallisation aus Essigester, Einengen der Mutterlauge und Chromatographie an Kieselgel (Fließmittel: Toluol/-Dioxan = 9:1). Ausbeute: 80%, Schmp.: 152-156°C.

### 4c. 1-Benzyl-7-benzyloxy-2-methyl-pyrrolo[2,3-d]pyridazin

650 mg (6 mMol) Benzylalkohol, 10 ml N-Methylpyrrolidon, 1,1 g (10 mMol) Kalium-tert.-butylat, 50 mg (0,2 mMol) [18]Krone-6 und 500 mg (1,94 mMol) 1-Benzyl-7-chlor-2-methyl-pyrrolo[2,3-d]pyridazin werden 2 h bei Raumtemperatur umgesetzt. Reinigung: Chromatographie an Kieselgel (Fließmittel: Toluol/Dioxan = 2:1) und Kristallisation aus Diisopropylether. Ausbeute: 68%, Schmp.: 124°C.

### 5a. 3-Amino-7-benzyloxy-1-isobutyl-2-methyl-pyrrolo[2,3-d]pyridazin-fumarat

Eine Suspension von 1,0 g (2,94 mMol) 7-Benzyloxy-1-isobutyl-2-methyl-3-nitro-pyrrolo[2,3-d]pyridazin in 30 ml Methanol wird mit einer Lösung von 2,6 g (14,6 mMol) Natriumdithionit in 30 ml Wasser versetzt und 30 Min. bei 70 °C gerührt. Nach Zugabe von 100 ml Wasser wird mit 3 x 100 ml Essigester extrahiert. Die organischen Extrakte werden mit 200 ml Wasser gewaschen, über Magnesiumsulfat getrocknet und anschließend mit einer Lösung von 341 mg (2,94 mMol) Fumarsäure in 10 ml Methanol versetzt. Anschließend wird die Lösung bis auf ein Volumen von 30 ml eingeengt. Nach Filtration werden 800 mg (64%) der Titelverbindung isoliert. Schmp.: 143°C (Zers.).

### 5b. 7-Benzyloxy-1-isobutyl-2-methyl-3-nitro-pyrrolo[2,3-d]pyridazin

Zu einer Lösung von 33,0 g (295 mMol) Kalium-tert.-Butylat und 1,5 g (5,9 mMol) [18]Krone-6 in 180 ml wasserfreiem N-Methylpyrrolidon werden 18,3 ml (177 mMol) Benzylalkohol bei Raumtemperatur zugetropft. Anschliessend wird noch 1 h bei Raumtemperatur gerührt. Danach wird eine Lösung von 15,9 g (59 mMol) 7-Chlor-1-isobutyl-2-methyl-3-nitro-pyrrolo[2,3-d]pyridazin in 50 ml wasserfreiem N-Methylpyrrolidon während 15 Min. zugetropft. Die Suspension wird dann 2 h bei 80°C gerührt. Nach Abkühlen auf Raumtemperatur wird mit 400 ml Eiswasser versetzt, und mit 3 x 300 ml Essigester extrahiert. Die organischen Extrakte werden mit 3 x 200 ml Wasser gewaschen und anschließend über Magnesiumsulfat getrocknet. Der nach Einengen verbleibende Rückstand wird an Kieselgel (Fließmittel: Toluol/Essigester = 4:1) chromatographiert. Nach Einengen der Fraktionen mit Rf = 0,25 und Kristallisation aus Diisopropylether/Essigester werden 5,1 g (25%) der Titelverbindung isoliert. Schmp.: 162-164°C.

### 5c. 7-Chlor-1-isobutyl-2-methyl-3-nitro-pyrrolo[2,3-d]pyridazin

16,0 g (71,5 mMol) 7-Chlor-1-isobutyl-2-methyl-pyrrolo[2,3-d]pyridazin werden bei 0°C unter starkem Rühren portionsweise in einem Gemisch von 100 ml rauchender Salpetersäure und 50 ml konz. Schwefelsäure gelöst. Die Lösung wird noch 4h bei Raumtemperatur gerührt und anschließend in 400 ml Eiswasser eingetragen. Danach wird mit 10 N Natronlauge neutralisiert und mit 3 x 250 ml Essigester extrahiert. Die organischen Extrakte werden über Magnesiumsulfat getrocknet und eingeengt. Nach Kristallisation aus Diisopropylether/Essigester werden 15,9 g (82%) der Titelverbindung isoliert. Schmp.: 96°C (Zers.).

### 6. 1-Benzyl-7-benzylamino-3-brom-2-methyl-pyrrolo[2,3-d]pyridazin

Eine Lösung von 1,0 g (2,97 mmol) 1-Benzyl-3-brom-7-chlor-2-methyl-pyrrolo-[2,3-d]pyridazin in 5 ml Benzylamin wird 5 h auf 150°C erhitzt und anschließend auf Raumtemperatur abgekühlt. Die Lösung wird dann mit 50 ml Wasser versetzt, mit 2N Salzsäure auf pH 6 gestellt und mit 2 x 50 ml Dichlormethan extrahiert. Die organischen Extrakte werden über Magnesiumsulfat getrocknet und eingeengt. Der verbleibende Rückstand wird an Kieselgel (Fließmittel: Toluol/Dioxan = 2:1) chromatographiert. Nach Einengen der Fraktionen mit Rf = 0,25 und Kristallisation aus Diisopropylether werden 1,01 (84%) der Titelverbindung isoliert. Schmp.: 185-188°C.

### 7a. 3-Acetoxymethyl-1-benzyl-7-benzyloxy-2-methyl-pyrrolo[2,3-d]pyridazin

Eine Lösung von 1,65 g (4,59 mmol) 1-Benzyl-7-benzyloxy-3-hydroxymethyl-2-methyl-pyrrolo[2,3-d]pyridazin, 3,8 g (18,6 mmol) Dicyclohexylcarbodiimid und 1,6 ml (27,4 mmol) Eisessig in 50 ml wasserfreiem Tetrahydrofuran wird 18 h bei Raumtemperatur gerührt. Die Lösung wird dann mit 50 ml Wasser versetzt, mit gesättigter Natriumhydrogencarbonatlösung neutral gestellt und mit 3 x 50 ml Dichlormethan extrahiert. Die organischen Extrakte werden mit 50 ml Wasser gewaschen, über Magnesiumsulfat getrocknet und eingeengt. Der verbleibende Rückstand wird an Kieselgel (Fließmittel: Essigester) chromatographiert. Nach Kristallisation aus Diisopropylether werden 1,3 g (71%) der Titelverbindung isoliert. Schmp.: 121-122°C.

### 7b. 3-Acetoxymethyl-1-benzyl-7-(4-fluorbenzyloxy)-2-methyl-pyrrolo[2,3-d]-pyridazin

0,1 g (0,26 mmol) 1-Benzyl-7-(4-fluorbenzyloxy)-3-hydroxymethyl-2-methyl-pyrrolo[2,3-d]pyridazin, 0,164 g (0,8 mmol) Dicyclohexylcarbodiimid und 0,046 ml (0,8 mmol) Eisessig werden in 5 ml wasserfreiem Tetrahydrofuran, wie für Beispiel 7a. beschrieben, umgesetzt. Ausbeute: 55%, Schmp.: 109-110°C.

### 7c. 1-Benzyl-7-(4-fluorbenzyloxy)-3-methoxyacetoxymethyl-2-methyl-pyrrolo-[2,3-d]pyridazin

0,1 g (0,25 mmol) 1-Benzyl-7-(4-fluorbenzyloxy)-3-hydroxymethyl-2-methyl-pyrrolo[2,3-d]pyridazin, 0,164 g (0,8 mmol) Dicyclohexylcarbodiimid und 0,061 ml (0,8 mmol) Methoxyessigsäure werden in 5 ml wasserfreiem Tetrahydrofuran, wie für Beispiel 7a. beschrieben, umgesetzt. Ausbeute: 60%, Schmp.: 119-121°C.

### 8. Bernsteinsäure-mono-[(1-benzyl-7-benzyloxy-2-methyl-pyrrolo[2,3-d]-pyridazin-3-yl)-methyl]-ester

Eine Lösung von 0,2 g (0,55 mmol) 1-Benzyl-7-benzyloxy-3-hydroxymethyl-2-methyl-pyrrolo[2,3-d]pyridazin und 72 mg (0,72 mmol) Bernsteinsäureanhydrid in 10 ml wasserfreiem Tetrahydrofuran wird 5 h am Rückfluß erhitzt. Nach Abkühlen auf Raumtemperatur wird der Niederschlag abgesaugt, mit Essigester nachgewaschen und im Hochvakuum getrocknet. 0,12 g (48%) der Titelverbindung werden isoliert. Schmp.: 189-190°C.

### Ausgangsverbindungen

### Aa. 1-Benzyl-7-chlor-2-methyl-pyrrolo[2,3-d]pyridazin

Eine Suspension von 2,0 g (8,3 mMol) 1-Benzyl-2-methyl-6,7-dihydro-pyrrolo-[2,3-d]pyridazin-7-on in 20 ml Phosphoroxychlorid wird 7 h bei 85°C gerührt. Anschließend wird das überschüssige Phosphoroxychlorid abdestilliert und der Rückstand in 50 ml Wasser hydrolysiert. Nach Neutralisation mit 2N Natronlauge wird mit 3 x 50 ml Dichlormethan extrahiert. Die organischen Extrakte werden mit 50 ml Wasser gewaschen, über Magnesiumsulfat getrocknet und eingeengt. Der Rückstand wird aus Methanol umkristallisiert. 1,8 g (84%) der Titelverbindung werden isoliert. Schmp.: 152-156°C.

Auf analoge Weise werden hergestellt:

### Ab. 7-Chlor-1-(4-methoxybenzyl)-2-methyl-pirrolo[2,3-d]pyridazin

17,8 g (66 mMol) 1-(4-Methoxybenzyl)-2-methyl-6,7-dihydro-pyrrolo[2,3-d]-pyridazin-7-on werden in 130 ml Phosphoroxychlorid 2 h bei 100°C umgesetzt. Reinigung: Chromatographie an Kieselgel (Fließmittel: Essigester). Ausbeute: 76%. Schmp.: 126-128°C.

### Ac. 7-Chlor-1-isobutyl-2-methyl-pyrrolo[2,3-d]pyridazin

3,8 g (12 mMol) 1-Isobutyl-2-methyl-6,7-dihydro-pyrrolo[2,3-d]pyridazin werden in 30 ml Phosphoroxychlorid 2 h bei 100 °C umgesetzt. Reinigung: Chromatographie an Kieselgel (Fließmittel: Toluol/Dioxan = 2:1). Ausbeute: 75%. Schmp.: 96-99°C.

### Ba. 1-Benzyl-2-methyl-6,7-dihydro-pyrrolo[2,3-d]pyridazin-7-on

Eine Lösung von 8,0 g (22 mMol) 1-Benzyl-7-benzyloxy-4-chlor-2-methyl-pyrrolo[2,3-d]pyridazin (verunreinigt mit ca. 5% der isomeren 4-Benzyloxy-Verbindung) und 5 ml Triethylamin in 800 ml Methanol wird 3h an 0,8 g Palladium(5%)/Kohle-Katalysator mit Wasserstoff hydriert. Anschließend wird vom Katalysator abfiltriert und bis auf 100 ml eingeengt. Nach Zugabe von 20 ml Essigester und kräftigem Ausrühren wird das Produkt abfiltriert und getrocknet. 3,4 g (65%) der Titelverbindung werden isoliert. Schmp.: 228-230°C. Das isomere Pyridazin-4-on bleibt in der Mutterlauge zurück.

Auf analoge Weise werden hergestellt:

### Bb. 1-(4-Methoxybenzyl)-2-methyl-6,7-dihydro-pyrrolo[2,3-d]pyridazin-7-on

38,3 g (97 mMol) 7-Benzyloxy-4-chlor-1-(4-methoxybenzyl)-2-methyl-pyrrolo-[2,3-d]pyridazin (verunreinigt mit ca. 5% der isomeren 4-Benzyloxy-Verbindung), 15 ml Triethylamin und 3,0 g Palladium(5%)/Kohle-Katalysator werden in 3 1 Methanol 10 h mit Wasserstoff hydriert. Reinigung: Kristallisation aus Methanol. Ausbeute: 69%, Schmp.: 212-213°C.

### Bc. 1-Isobutyl-2-methyl-6,7-dihydro-pyrrolo[2,3-d]pyridazin-7-on

4,5 g (13,6 mMol) 7-Benzyloxy-4-chlor-1-isobutyl-2-methyl-pyrrolo[2,3-d]pyridazin (verunreinigt mit ca. 40% der isomeren 4-Benzyloxy-Verbindung), 4 ml Triethylamin und 0,5 g Palladium(5%)/Kohle-Katalysator werden in 500 ml Methanol 5 h mit Wasserstoff hydriert. Reinigung: Chromatographie an Kieselgel (Fließmittel: Toluol/Dioxan 4:1). Ausbeute: 52%, Schmp.: 188-191°C.

### Ca. 1-Benzyl-7-benzyloxy-4-chlor-2-methyl-pyrrolo[2,3-d]pyridazin und 1-Benzyl-4-benzyloxy-7-chlor-2-methyl-pyrrolo[2,3-d]puridazin

Eine Lösung von 4,3 g (39,8 mMol) Benzylalkohol in 10 ml wasserfreiem Tetrahydrofuran wird während 10 Min. zu einer Suspension von 1,2 g 80%igem Natriumhydrid (42 mMol) in 25 ml wasserfreiem Tetrahydrofuran getropft. Die Mischung wird noch 1 h gerührt und anschließend bei 20 °C langsam zu einer Lösung von 11,2 g (38,3 mMol) 1-Benzyl-4,7-dichlor-2-methyl-pyrrolo[2,3-d]-pyridazin in 50 ml wasserfreiem Tetrahydrofuran getropft. Die Lösung wird noch 30 Min. bei 20°C gerührt, anschließend mit 150 ml Wasser versetzt und mit 3 x 100 ml Essigester extrahiert. Die organischen Extrakte werden mit 150 ml Wasser gewaschen, über Magnesiumsulfat getrocknet und eingeengt.

Ausbeute: 81%. Die Isomeren werden nicht getrennt; das Gemisch wird direkt für die nächste Synthesestufe eingesetzt (siehe Beispiel Ba.).

Auf analoge Weise werden hergestellt:

### Cb. 7-Benzyloxy-4-chlor-1-(4-methoxybenzyl)-2-methyl-pyrrolo[2,3-d]pyridazin und 4-Benzyloxy-7-chlor-1-(4-methoxybenzyl)-2-methyl-pyrrolo-[2,3-d]pyridazin

4,28 g (149 mMol) Natriumhydrid (80%ig), 16,0 g (149 mMol) Benzylalkohol und 30,0 g (93 mMol) 4,7-Dichlor-1-(4-methoxybenzyl)-2-methyl-pyrrolo-[2,3-d]pyridazin werden in insgesamt 150 ml wasserfreiem Tetrahydrofuran umgesetzt. Die Isomeren werden nicht getrennt; das Gemisch wird direkt für die nächste Synthesestufe eingesetzt (siehe Beispiel Bb.).

### Cc. 7-Benzyloxy-4-chlor-1-isobutyl-2-methyl-pyrrolo[2,3-d]pyridazin und 4-Benzyloxy-7-chlor-1-isobutyl-2-methyl-pyrrolo[2,3-d]pyridazin

1,2 g (40 mMol) Natriumhydrid (80%ig), 4,4 g (40 mMol) Benzylalkohol und 7,0 g (27 mMol) 4,7-Dichlor-1-isobutyl-2-methyl-pyrrolo[2,3-d]pyridazin werden in insgesamt 80 ml wasserfreiem Tetrahydrofuran umgesetzt. Die Isomeren werden nicht getrennt; das Gemisch wird direkt für die nächste Synthesestufe eingesetzt (siehe Beispiel Bc.).

### Da. 1-Benzyl-4,7-dichlor-2-methyl-pyrrolo[2,3-d]pyridazin

Eine Suspension von 6,2 g (24 mMol) 1-Benzyl-2-methyl-4,5,6,7-tetrahydropyrrolo[2,3-d]pyridazin-4,7-dion in 30 ml Phosphoroxychlorid wird 2 h am Rückfluß erhitzt. Anschließend wird das überschüssige Phosphoroxychlorid abdestilliert und der Rückstand in 100 ml Wasser aufgenommen. Nach Neutralisation mit 10 N Natronlauge wird mit 3 x 100 ml Essigester extrahiert. Die organischen Extrakte werden mit 150 ml Wasser gewaschen, über Magnesiumsulfat getrocknet und eingeengt. Der Rückstand wird in 300 ml Toluol/Dioxan (9:1) aufgenommen und mit Kieselgel geklärt. Nach Einengen erhält man 5,69 g (80%) der Titelverbindung. Schmp.: 122-124°C.

Auf analoge Weise werden hergestellt:

### Db. 4,7-Dichlor-1-(4-methoxybenzyl)-2-methyl-pyrrolo[2,3-d]pyridazin

33,0 g (120 mMol) 1-(4-methoxybenzyl)-2-methyl-4,5,6,7-tetrahydro-pyrrolo-[2,3-d]pyridazin-4,7-dion werden in 175 ml Phosphoroxychlorid 2 h am Rückfluß erhitzt. Reinigung: Kristallisation aus Essigester/Diisopropylether. Ausbeute: 80%, Schmp.: 120-123°C.

### Dc. 4,7-Dichlor-1-isobutyl-2-methyl-pyrrolo[2,3-d]pyridazin

7,0 g (31,6 mMol) 1-Isobutyl-2-methyl-4,5,6,7-tetrahydro-pyrrolo[2,3-d]-pyridazin-4,7-dion werden in 50 ml Phosphoroxychlorid 2 h am Rückfluß erhitzt. Reinigung: Chromatographie an Kieselgel (Fließmittel: Toluol/Dioxan = 9:1). Ausbeute: 88%, Schmp.: 89°C (Zers.).

### Ea. 1-Benzyl-2-methyl-4,5,6,7-tetrahydro-pyrrolo[2,3-d]pyridazin-4,7-dion

Eine Lösung von 175,3 g (0,56 Mol) 1-Benzyl-5-methyl-pyrrol-2,3-dicarbonsäurediethylester und 540 ml (11,1 Mol) Hydrazinhydrat in 880 ml Diglyme wird auf 110°C erhitzt. Nach 2 h werden nochmals 270 ml (5,5 Mol) Hydrazinhydrat während 90 min. zugetropft. Anschließend wird noch 16 h bei 110°C gerührt. Nach Abkühlen und Zugabe von 2 l Eiswasser wird mit konz. Salzsäure auf pH 5 gestellt. Der Niederschlag wird abgesaugt und mit 5 l Wasser nachgewaschen. Nach Trocknen über Kaliumhydroxid im Hochvakuum und Ausrühren aus Diisopropylether/Essigester werden 127,3 g (89%) der Titelverbindung isoliert. Schmp.: 322-324°C.

Auf analoge Weise werden hergestellt:

### Eb. 1-(4-methoxybenzyl)-2-methyl-4,5,6,7-tetrahydro-pyrrolo[2,3-d]-pyridazin-4,7-dion

46,0 g (0,133 Mol) 1-(4-Methoxybenzyl)-5-methyl-pyrrol-2,3-dicarbonsäurediethylester und 129,5 ml (2,66 Mol) Hydrazinhydrat werden in 290 ml Diglyme 18 h bei 110°C umgesetzt. Reinigung: Ausrühren aus Diisopropylether/Essigester. Ausbeute: 87%, Schmp.: 300°C.

### Ec. 1-Isobutyl-2-methyl-4,5,6,7-tetrahydro-pyrrolo[2,3-d]pyridazin-4,7-dion

9,0 g (31,9 mMol) 1-Isobutyl-5-methyl-pyrrol-2,3-dicarbonsäurediethylester und 48 ml (985 mMol) Hydrazinhydrat werden in 50 ml Diglyme 18 h bei 110°C umgesetzt. Reinigung: Ausrühren aus Diisopropylether/Essigester. Ausbeute: 62%, Schmp.: 278-282°C.

### Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgeprägte Magensäuresekretionshemmung und eine ausgezeichnete Magen- und Darmschutzwirkung bei Warmblütern auf. Darüberhinaus zeichnen sich die erfindungsgemäßen Verbindungen durch eine hohe Wirkungsselektivität, das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen (z.B. Helicobacter pylori), Bakterientoxine, Medikamente (z.B. bestimmte Antiphlogistika und Antirheumatika), Chemikalien (z.B. Ethanol), Magensäure oder Streßsituationen verursacht werden können.

In ihren ausgezeichneten Eigenschaften erweisen sich die erfindungsgemäßen Verbindungen an verschiedenen Modellen, in denen die antiulcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und/oder Prophylaxe von Erkrankungen des Magens und/oder Darms verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsoder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Könpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise H₂-Blockern (z.B. Cimetidin, Ranitidin), oder ferner mit sogenannten peripheren Anticholinergika (z.B. Pirenzepin, Telenzepin) sowie mit Gastrin-Antagonisten mit dem Ziel, die Hauptwirkung in additivem oder überadditivem Sinn zu verstärken und/oder die Nebenwirkungen zu eliminieren oder zu verringern, oder ferner die Kombination mit antibakteriell wirksamen Substanzen (wie z.B. Cephalosporinen, Tetracyclinen, Nalidixinsäure, Penicillinen oder auch Wismutsalzen) zur Bekämpfung von Helicobacter pylori.

### Pharmakologie

Die ausgezeichnete Magenschutzwirkung und die magensekretionshemmende Wirkung der erfindungsgemäßen Verbindungen kann in Untersuchungen an tierexperimentellen Modellen nachgewiesen werden. Die in dem nachstehend aufgeführten Modell untersuchten erfindungsgemäßen Verbindungen sind mit Nummern versehen worden, die den Nummern dieser Verbindungen in den Beispielen entsprechen.

### Prüfung der sekretionshemmenden Wirkung am perfundierten Rattenmagen

In der folgenden Tabelle 2 ist der Einfluß der erfindungsgemäßen Verbindungen nach intravenöser Gabe auf die durch Pentagastrin stimulierte Säuresekretion des perfundierten Rattenmagens in vivo dargestellt.

**Tabelle 2**

| Nr. | N (Anzahl der Tiere) | Dosis (µmol/kg) i.v. | Maximale Hemmung der Säureausscheidung ca. ED50 +) (µmol/kg) | |
|---|---|---|---|---|
| | | | (%) | i.v. |
| 1a | 3 | 0,3 | 22 | 1,1 |
| | 3 | 1,0 | 52 | |
| | 3 | 3,0 | 70 | |
| | 3 | 10,0 | 94 | |
| +) ED50 = Dosis (interpoliert), die eine maximale Hemmung der HCl-Sekretion um 50 % bewirkt. | | | | |

### Methodik

Narkotisierten Ratten (CD-Ratte, weiblich, 200-250 g; 1,5 g/kg i.m. Urethan) wurde nach Tracheotomie das Abdomen durch einen medianen Oberbauchschnitt eröffnet und ein PVC-Katheter transoral im Ösophagus sowie ein weiterer via Pylorus derart fixiert, daß die Schlauchenden eben noch in das Magenlumen hineinragten. Der aus dem Pylorus führende Katheter führte über eine seitliche Öffnung in der rechten Bauchwand nach außen.

Nach gründlicher Spülung (ca. 50-100 ml) wurde der Magen mit 37°C warmer physiologischer NaCl-Lösung kontinuierlich durchströmt (0,5 ml/min, pH 6,8-6,9; Brun-Unita I). In dem jeweils im 15 Min.-Abstand aufgefangenen (25 ml Meßzylinder) Effluat wurde der pH-Wert (pH-Meter 632, Glaselektrode EA 147; φ = 5 mm, Metrohm) sowie durch Titration mit einer frisch zubereiteten 0,01 N NaOH bis pH 7 (Dosimat 655 Metrohm) die sezernierte HCl bestimmt.

Die Stimulation der Magensekretion erfolgte durch Dauerinfusion von 1 µg/kg (= 1,65 ml/h) i.v. Pentagastrin (V. fem. sin.) ca. 30 Min. nach Operationsende (d.h. nach Bestimmung von 2 Vorfraktionen). Die zu prüfenden Substanzen wurden intravenös (V. jugularis sin.) in 1 ml/kg Flüssigkeitsvolumen 60 Min. nach Beginn der Pentagastrin-Dauerinfusion verabreicht.

Die Körpertemperatur der Tiere wurde durch Infrarot-Bestrahlung und Heizkissen (automatische, stufenlose Regelung über rektalen Temperaturfühler) auf konstant 37,8 - 38°C gehalten.

Als Maß für die sekretionshemmende Wirkung diente die maximale Abnahme der Säureausscheidung (15 Min.-Fraktionen) jeder behandelten Gruppe gegenüber derjenigen der unbehandelten Kontrollgruppe (= 100 %). Die ED50 bezeichnet diejenige Dosis, die eine maximale Hemmung der HCl-Sekretion um 50 % bewirkt.

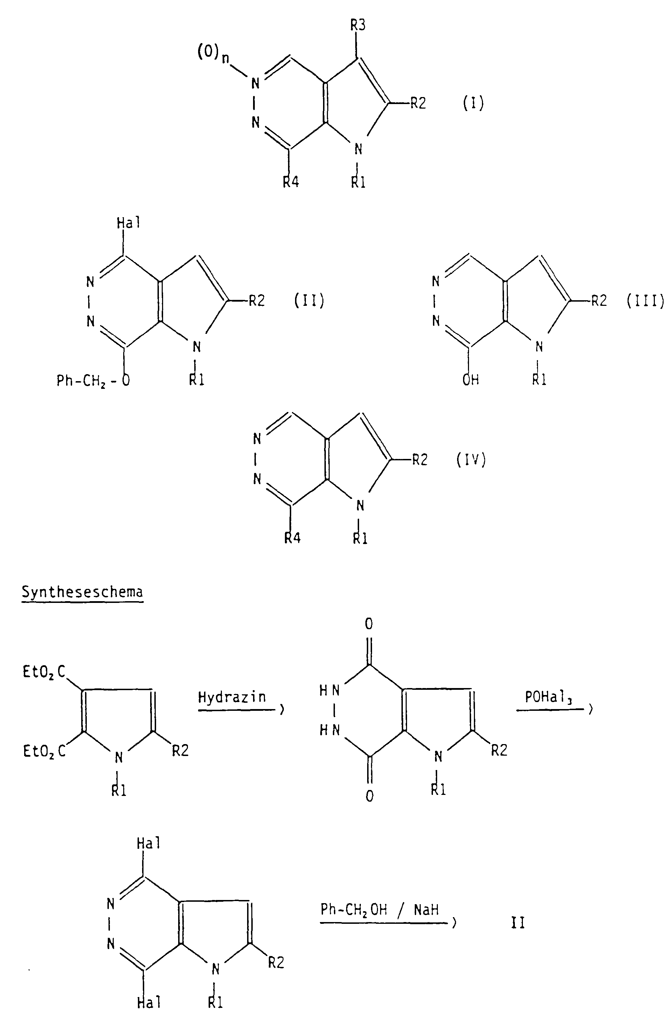

## Patentansprüche

1. Verbindungen der Formel I 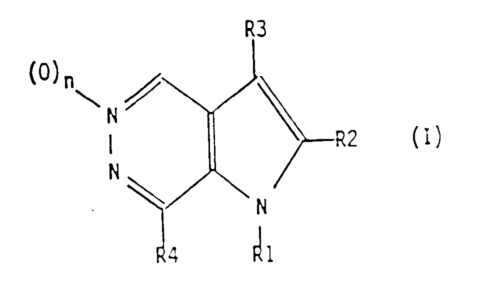 worin
R1 1-4C-Alkyl oder durch R5 substituiertes 1-3C-Alkylen,
R2 1-4C-Alkyl,
R3 Wasserstoff, Halogen, CHO (Formyl), Hydroxymethyl, Nitro, Amino oder den Substituenten -CH₂O-COR7,
R4 Halogen oder den Substituenten -A-B-R6,
R5 Furyl, Thienyl, Tetrahydrofuryl, Phenyl oder durch einen oder zwei gleiche oder verschiedene Substituenten aus der Gruppe Halogen, 1-4C-Alkyl und 1-4C-Alkoxy substituiertes Phenyl,
R6 Wasserstoff, Thienyl, Furyl, Phenyl oder durch einen oder zwei gleiche oder verschiedene Substituenten aus der Gruppe Halogen, 1-4C-Alkyl, 1-4C-Alkoxy, Nitro, -NH-CO-NH₂ (Ureido), Amino, 1-4C-Alkylcarbonylamino und 1-4C-Alkoxycarbonylamino substituiertes Phenyl,
R7 1-4C-Alkyl, 1-4C-Alkoxy-1-4C-alkyl, 1-4C-Alkoxycarbonyl-1-4C-alkyl oder Carboxy-1-4C-alkyl,
A 0 (Sauerstoff) oder NH,
B einen Bindungsstrich, -CH₂- (Methylen) oder -CH₂CH₂- (1,2-Ethylen) und
n die Zahl 0 oder 1 darstellt,
und die Salze dieser Verbindungen.

2. Verbindungen der Formel I nach Anspruch 1, worin R3 Wasserstoff, R6 Wasserstoff, B einen Bindungsstrich und n die Zahl 0 darstellt, und R1, R2, R4, R5 und A die in Anspruch 1 angegebenen Bedeutungen haben, und ihre Salze.

3. Verbindungen der Formel I nach Anspruch 1, worin
R1 1-4C-Alkyl oder durch R5 substituiertes 1-3C-Alkylen,
R2 1-4C-Alkyl,
R3 Hydroxymethyl, Nitro, Amino oder den Substituenten -CH₂O-COR7,
R4 den Substituenten -A-B-R6,
R5 Furyl, Thienyl, Tetrahydrofuryl, Phenyl oder durch einen oder zwei gleiche oder verschiedene Substituenten aus der Gruppe Halogen, 1-4C-Alkyl und 1-4C-Alkoxy substituiertes Phenyl,
R6 Thienyl, Furyl, Phenyl oder durch einen oder zwei gleiche oder verschiedene Substituenten aus der Gruppe Halogen, 1-4C-Alkyl, 1-4C-Alkoxy, Nitro, -NH-CO-NH₂ (Ureido), Amino, 1-4C-Alkylcarbonylamino und 1-4C-Alkoxycarbonylamino substituiertes Phenyl,
R7 1-4C-Alkyl, 1-4C-Alkoxy-1-4C-alkyl, 1-4C-Alkoxycarbonyl-1-4C-alkyl oder Carboxy-1-4C-alkyl,
A 0 (Sauerstoff) oder NH,
B -CH₂- (Methylen) oder -CH₂CH₂- (1,2-Ethylen) und
n die Zahl 0 oder 1 darstellt,
und die Salze dieser Verbindungen.

4. Verbindungen der Formel I nach Anspruch 1, worin
R1 Isobutyl oder durch R5 substituiertes Methylen,
R2 1-4C-Alkyl
R3 Hydroxymethyl, Nitro, Amino oder den Substituenten -CH₂O-COR7,
R4 den Substituenten -A-B-R6,
R5 Furyl, Thienyl, Phenyl oder durch einen oder zwei gleiche oder verschiedene Substituenten aus der Gruppe Halogen, 1-4C-Alkyl und 1-4C-Alkoxy substituiertes Phenyl,
R6 Thienyl, Phenyl oder durch einen oder zwei gleiche oder verschiedene Substituenten aus der Gruppe Halogen, 1-4C-Alkyl, 1-4C-Alkoxy, -NH-CO-NH₂ (Ureido), Amino, 1-4C-Alkylcarbonylamino und 1-4C-Alkoxycarbonylamino substituiertes Phenyl,
R7 1-4C-Alkyl, 1-4C-Alkoxy-1-4C-alkyl, 1-4C-Alkoxycarbonyl-1-4C-alkyl oder Carboxy-1-4C-alkyl,
A 0 (Sauerstoff) oder NH,
B -CH₂- (Methylen) und
n die Zahl 0 oder 1 darstellt,
und die Salze dieser Verbindungen.

5. Verbindungen der Formel I nach Anspruch 1, worin
R1 durch R5 substituiertes Methylen,
R2 1-4C-Alkyl,
R3 Hydroxymethyl, Amino oder den Substituenten -CH₂O-COR7,
R4 den Substituenten -A-B-R6,
R5 Phenyl oder durch einen Substituenten aus der Gruppe Chlor, Fluor und 1-4C-Alkoxy substituiertes Phenyl,
R6 Phenyl oder durch einen Substituenten aus der Gruppe Chlor und Fluor substituiertes Phenyl,
R7 Methyl , Methoxymethyl , Methoxycarbonylmethyl, Methoxycarbonylethyl Carboxymethyl oder Carboxyethyl,
A 0 (Sauerstoff),
B -CH₂- (Methylen) und
n die Zahl 0 darstellt,
und die Salze dieser Verbindungen.

6. 1-Benzyl-7-(4-fluorbenzyloxy)-3-hydroxymethyl-2-methyl-pyrrolo-[2,3-d]-pyridazin, und die Salze dieser Verbindung.

7. Verfahren zur Herstellung der Verbindungen der Formel I nach Anspruch I, und ihrer Salze, dadurch gekennzeichnet, daß man
a) zur Herstellung von Verbindungen I, in denen R3 und R6 Wasserstoff, R4 den Substituenten -A-B-R6, A Sauerstoff, B einen Bindungsstrich und n die Zahl 0 bedeutet, Verbindungen der Formel II 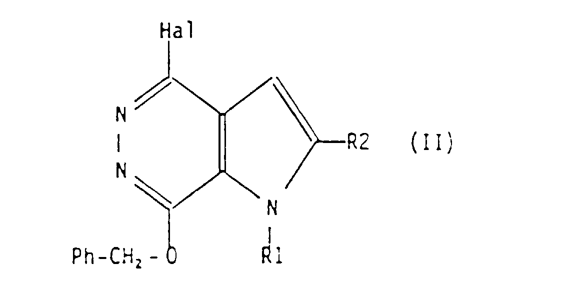 worin R1 und R2 die in Anspruch 1 angegebenen Bedeutungen haben und Hal ein Halogenatom, vorzugsweise ein Chloratom darstellt, reduktiv debenzyliert und enthalogeniert, oder daß man
b) zur Herstellung von Verbindungen I, in denen R3 Wasserstoff, R4 Halogen und n die Zahl 0 bedeutet, die gemäß a) erhaltenen Verbindungen der Formel III 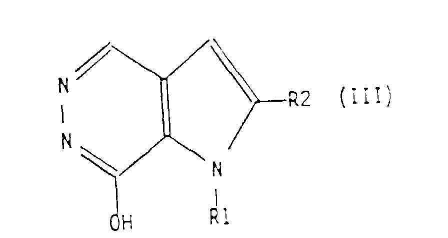 worin R1 und R2 die in Anspruch 1 angegebenen Bedeutungen haben, mit geeigneten Halogenierungsmitteln umsetzt, oder daß man
c) zur Herstellung von Verbindungen I, in denen R3 Wasserstoff, R4 den Substituenten -A-B-R6 und n die Zahl 0 bedeutet, die gemäß b) erhaltenen Verbindungen der Formel IV 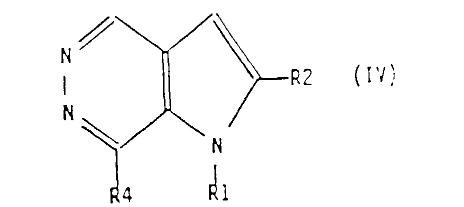 worin R4 Halogen bedeutet, mit Verbindungen der Formel H-A-B-R6 (= Verbindungen der Formel V), worin A, B und R6 die in Anspruch 1 angegebenen Bedeutungen haben, oder ihren Salzen mit Basen, umsetzt, oder daß man
d) zur Herstellung von Verbindungen I, in denen R3 Halogen und n die Zahl 0 bedeutet, Verbindungen der Formel IV, worin R1, R2 und R4 die in Anspruch 1 angegebenen Bedeutungen haben, mit geeigneten Halogenierungsmitteln umsetzt, oder daß man
e) zur Herstellung von Verbindungen I, in denen R3 CHO (Formyl) und n die Zahl 0 bedeutet, Verbindungen der Formel I, worin R3 Halogen bedeutet, in 3-Position intermediär metalliert und anschließend mit Dimethylformamid oder Ameisensäureester umsetzt, oder daß man
f) zur Herstellung von Verbindungen I, in denen R3 Hydroxymethyl und n die Zahl 0 bedeutet, Verbindungen der Formel I, worin R3 CHO (Formyl) bedeutet, reduziert, oder daß man
g) zur Herstellung von Verbindungen I, in denen n die Zahl 1 bedeutet, Verbindungen der Formel I, worin n die Zahl 0 bedeutet, oxydiert, oder daß man
h) zur Herstellung von Verbindungen I, in denen R4 den Rest -A-B-R6 bedeutet, wobei R6 durch 1-4C-Alkoxycarbonylamino oder 1-4C-Alkylcarbonylamino substituiertes Phenyl bedeutet, Verbindungen der Formel I, worin R4 den Rest -A-B-R6 bedeutet, wobei R6 durch Amino substituiertes Phenyl bedeutet, mit Halogenameisensäure-1-4C-alkylester bzw. mit 1-4C-Carbonsäurehalogenid umsetzt, oder daß man
i) zur Herstellung von Verbindungen I, in denen R3 Nitro und R4 Halogen bedeutet, Verbindungen der Formel I, worin R3 Wasserstoff und R4 Halogen bedeutet, nitriert, oder daß man
j) zur Herstellung von Verbindungen I, in denen R3 Nitro und R4 den Substituenten -A-B-R6 bedeutet, die gemäß i) erhaltenen Verbindungen der Formel I, worin R3 Nitro und R4 Halogen bedeutet, mit Verbindungen der Formel H-A-B-R6 (= Verbindungen der Formel V), worin A, B, und R6 die oben angegebenen Bedeutungen haben, oder ihren Salzen mit Basen, umsetzt, oder daß man
k) zur Herstellung von Verbindungen I, in denen R3 Amino und R4 den Substituenten -A-B-R6 bedeutet, die gemäß j) erhaltenen Verbindungen der Formel I, worin R3 Nitro und R4 den Substituenten -A-B-R6 bedeutet, reduziert, oder daß man
l) zur Herstellung von Verbindungen I, in denen R3 den Substituenten -CH₂O-COR7 und R7 1-4C-Alkyl, 1-4C-Alkoxy-1-4C-alkyl, 1-4C-Alkoxycarbonyl1-4C-alkyl oder Carboxy-1-4C-alkyl bedeutet, Verbindungen der Formel I, worin R3 Hydroxymethyl bedeutet, mit Verbindungen der Formel R7-CO-Z (= Verbindungen der Formel VI) umsetzt, worin R7 die vorstehenden Bedeutungen hat und Z OH (Hydroxy) oder eine geeignete Abgangsgruppe darstellt,
und daß man gewünschtenfalls anschließend die nach a), b), c), d), e), f), g), h), i), j), k) oder l) erhaltenen Verbindungen I in ihre Salze überführt, oder daß man gewünschtenfalls anschließend aus erhaltenen Salzen der Verbindungen I die Verbindungen I freisetzt.

8. Arzneimittel enthaltend eine oder mehrere Verbindungen nach einem oder mehreren der Ansprüche 1 bis 6 und/oder ihre pharmakologisch verträglichen Salze.

9. Verbindungen nach einem oder mehreren der Ansprüche 1 bis 6 und ihre pharmakologisch verträglichen Salze zur Anwendung bei der Verhütung und Behandlung gastrointestinaler Krankheiten.

10. Verwendung von Verbindungen nach einem oder mehreren der Ansprüche 1 bis 6 und ihren pharmakologisch verträglichen Salzen zur Herstellung von Arzneimitteln für die Verhütung und Behandlung gastrointestinaler Krankheiten.

## Claims

1. A compound of the formula I 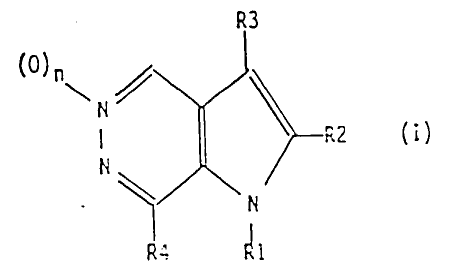 in which
R1 is 1-4C-alkyl or R5-substituted 1-3C-alkylene,
R2 is 1-4C-alkyl,
R3 is hydrogen, halogen, CHO (formyl), hydroxymethyl, nitro, amino or the substituent -CH₂O-COR7,
R4 is halogen or the substituent -A-B-R6,
R5 is furyl, thienyl, tetrahydrofuryl, phenyl or phenyl substituted by one or two identical or different substituents from the group consisting of halogen, 1-4C-alkyl and 1-4C-alkoxy,
R6 is hydrogen, thienyl, furyl, phenyl or phenyl substituted by one or two identical or different substituents from the group consisting of halogen, 1-4C-alkyl, 1-4C-alkoxy, nitro, -NH-CO-NH₂ (ureido), amino, 1-C4-alkylcarbonylamino and 1-4C-alkoxycarbonylamino,
R7 is 1-4C-alkyl, l-4C-alkoxy-1-4C-alkyl, 1-4C-alkoxycarbonyl-1-4C-alkyl or carboxy-1-4C-alkyl,
A is O (oxygen) or NH,
B is a valence bond, -CH₂- (methylene) or -CH₂CH₂-(1,2-ethylene) and
n is the number 0 or 1,
or the salts of this compound.

2. A compound of the formula I as claimed in claim 1, wherein R3 is hydrogen, R6 is hydrogen, B is a valence bond and n is the number 0, aid R1, R2, R4, R5 and A have the meanings indicated in claim 1, or its salts.

3. A compound of the formula I as claimed in claim 1, wherein
R1 is 1-4C-alkyl or R5-substituted 1-3C-alkylene,
R2 is 1-4C-alkyl,
R3 is hydroxymethyl, nitro, amino or the substituent -CH₂O-COR7,
R4 is the substituent -A-B-R6,
R5 is furyl, thienyl, tetrahydrofuryl, phenyl or phenyl substituted by one or two identical or different substituents from the group consisting of halogen, 1-4C-alkyl and 1-4C-alkoxy,
R6 is thienyl, furyl, phenyl or phenyl substituted by one or two identical or different substituents from the group consisting of halogen, 1-4C-alkyl, 1-4C-alkoxy, nitro, -NH-CO-NH₂ (ureido), amino, 1-4C-alkylcarbonylamino and 1-4C-alkoxycarbonylamino,
R7 is 1-4C-alkyl, 1-4C-alkoxy-1-4C-alkyl, 1-4C-alkoxycarbonyl-1-4C-alkyl or carboxy-1-4C-alkyl,
A is O (oxygen) or NH,
B is -CH₂- (methylene) or -CH₂CH₂- (1,2-ethylene) and
n is the number 0 or 1,
or the salts of this compound.

4. A compound of the formula I as claimed in claim 1, wherein
R1 is isobutyl or R5-substituted methylene,
R2 is 1-4C-alkyl,
R3 is hydroxymethyl, nitro, amino or the substituent -CH₂O-COR7,
R4 is the substituent -A-B-R6,
R5 is furyl, thienyl, phenyl or phenyl substituted by one or two identical or different substituents from the group consisting of halogen, 1-4C-alkyl and 1-4C-alkoxy,
R6 is thienyl, phenyl or phenyl substituted by one or two identical or different substituents from the group consisting of halogen, 1-4C-alkyl, 1-4C-alkoxy,-NH-CO-NH₂ (ureido), amino, 1-4C-alkylcarbonylamino and 1-4C-alkoxycarbonylamino,
R7 is 1-4C-alkyl, 1-4C-alkoxy-1-4C-alkyl, 1-4C-alkoxycarbonyl-1-4C-alkyl or carboxy-1-4C-alkyl,
A is O (oxygen) or NH,
B is -CH₂- (methylene) and
n is the number 0 or 1,
or the salts of this compound.

5. A compound of the formula I as claimed in claim 1, wherein
R1 is R5-substituted methylene,
R2 is 1-4C-alkyl,
R3 is hydroxymethyl, amino or the substituent -CH₂O-COR7,
R4 is the substituent -A-B-R6,
R5 is phenyl or phenyl substituted by a substituent from the group consisting of chlorine, fluorine and 1-4C-alkoxy,
R6 is phenyl or phenyl substituted by a substituent from the group consisting of chlorine and fluorine,
R7 is methyl, methoxymethyl, methoxycarbonylmethyl, methoxycarbonylethyl, carboxymethyl or carboxyethyl,
A is O (oxygen),
B is -CH₂- (methylene) and
n is the number 0,
or the salts of this compound.

6. 1-Benzyl-7-(4-fluorobenzyloxy)-3-hydroxymethyl-2-methylpyrrolo[2,3-d]pyridazine, and the salts of this compound.

7. A process for the preparation of the compounds of the formula I as claimed in claim 1, and their salts, which comprises
a) for the preparation of compounds I in which R3 and R6 denote hydrogen, R4 denotes the substituent - A-B-R6, A denotes oxygen, B denotes a valence bond and n denotes the number 0, reductively debenzylating and dehalogenating compounds of the formula II 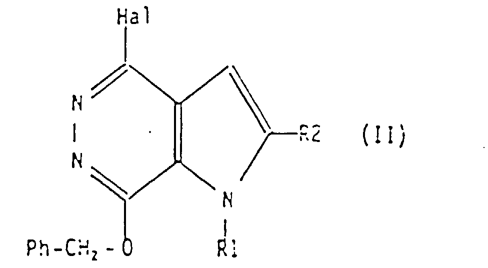 in which R1 and R2 have the meanings indicated in claim 1 and Hal is a halogen atom, preferably a chlorine atom, or
b) for the preparation of compounds I in which R3 denotes hydrogen, R4 denotes halogen and n denotes the number 0, reacting the compounds of the formula III 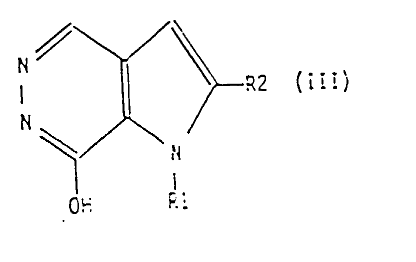 in which R1 and R2 have the meanings indicated in claim 1, obtained as in a) with suitable halogenating agents, or
c) for the preparation of compounds I in which R3 denotes hydrogen, R4 denotes the substituent -A-B-R6 and n denotes the number 0, reacting the compounds of the formula IV 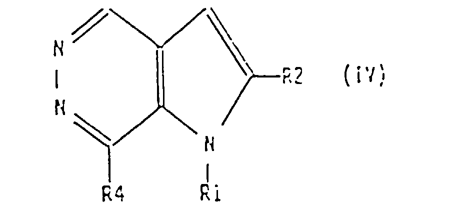 in which R4 denotes halogen, obtained as in b) with compounds of the formula H-A-B-R6 (= compounds of the formula V), in which A, B and R6 have the meanings indicated in claim 1, or their salts with bases, or
d) for the preparation of compounds I in which R3 denotes halogen and n denotes the number 0, reacting compounds of the formula IV, in which R1, R2 and R4 have the meanings indicated in claim 1, with suitable halogenating agents, or
e) for the preparation of compounds I in which R3 denotes CHO (formyl) and n denotes the number 0, intermediately metalating compounds of the formula I, in which R3 denotes halogen, in the 3-position and subsequently reacting with dimethylformamide or formic acid esters, or
f) for the preparation of compounds I in which R3 denotes hydroxymethyl and n denotes the number 0, reducing compounds of the formula I in which R3 denotes CHO (formyl), or
g) for the preparation of compounds I in which n denotes the number 1, oxidizing compounds of the formula I in which n denotes the number 0, or
h) for the preparation of compounds I in which R4 denotes the radical -A-B-R6, where R6 denotes phenyl substituted by 1-4C-alkoxycarbonyla%ino or 1-4C-alkylcarbonylamino, reacting compounds of the formula I, in which R4 denotes the radical -A-B-R6, where R6 denotes phenyl substituted by amino, with 1-4C-alkyl haloformates or with 1-4C-carboxylic acid halide, or
i) for the preparation of compounds I in which R3 denotes nitro and R4 denotes halogen, nitrating compounds of the formula I in which R3 denotes hydrogen and R4 denotes halogen, or
j) for the preparation of compounds I in which R3 denotes nitro and R4 denotes the substituent -A-B-R6, reacting the compounds of the formula I, in which R3 denotes nitro and R4 denotes halogen, obtained as in i) with compounds of the formula H-A-B-R6 (= compounds of the formula V), in which A, B and R6 have the meanings indicated above, or their salts with bases, or
k) for the preparation of compounds I in which R3 denotes amino and R4 denotes the substituent -A-B-R6, reducing the compounds of the formula I, in which R3 denotes nitro and R4 denotes the substituent -A-B-R6, obtained as in j), or
l) for the preparation of compounds I in which R3 denotes the substituent -CH₂O-COR7 and R7 denotes 1-4C-alkyl, 1-4C-alkoxy-1-4C-alkyl, 1-4C-alkoxycarbonyl-1-4C-alkyl or carboxy-1-4C-alkyl, reacting compounds of the formula I in which R3 denotes hydroxymethyl with compounds of the formula R7-CO-Z (= compounds of the formula VI) in which R7 has the above meanings and Z is OH (hydroxyl) or a suitable leaving group,
and, if desired, subsequently converting the compounds I obtained according to a), b), c), d), e), f), g), h), i), j), k) or l) into their salts, or, if desired, subsequently liberating the compounds I from salts of the compounds I obtained.

8. A medicament containing one or more compounds as claimed in one or more of claims 1 to 6 and/or their pharmacologically tolerable salts.

9. A compound as claimed in one or more of claims 1 to 6 and its pharmacologically tolerable salts for use in the prevention and treatment of gastrointestinal diseases.

10. The use of compounds as claimed in one or more of claims 1 to 6 and their pharmacologically tolerable salts for the production of medicaments for the prevention and treatment of gastrointestinal diseases.

## Revendications

1. Composés de formule I 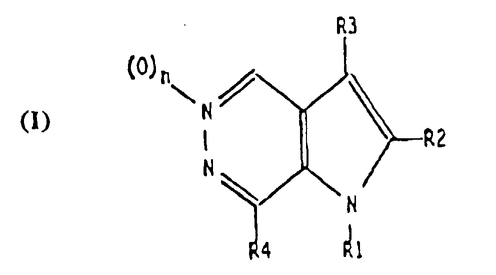 dans lesquels
R¹ représente un groupe alkylc en C₁₋₄ ou un groupe alkylène en C₁₋₃ portant un substituant R⁵,
R² représente un groupe alkyle en C₁₋₄,
R³ représente un atome d'hydrogène ou d'halogène, un groupe CHO (formyle), hydroxyméthyle, nitro, amino ou le substituant -CH₂O-COR⁷,
R⁴ représente un atome d'halogène ou le substituant -A-B-R⁶,
R⁵ représente un groupe furyle, thiényle, tétrahydrofuryle, phényle ou phényle portant un ou deux substituants, identiques ou différents, choisis dans le groupe formé par un atome d'halogène, un groupe alkyle en C₁₋₄ et alcoxy en C₁₋₄,
R⁶ représente un atome d'hydrogène, un groupe thiényle, furyle, phényle ou phényle portant un ou deux substituants, identiques ou différents, choisis dans le groupe formé par un atome d'halogène, un groupe alkyle en C₁₋₄, alcoxy en C₁₋₄, nitro, -NH-CO-NH₂ (uréido), amino, (alkyle en C₁₋₄)-carbonylamino et (alcoxy en C₁₋₄)-carbonylamino,
R⁷ représente un groupe alkyle en C₁₋₄, (alcoxy en C₁₋₄)-(alkyle en C₁₋₄), (alcoxy en C₁₋₄)-carbonyl-(alkyle en C₁₋₄) ou carboxy-(alkyle en C₁₋₄),
A représente un atome d'oxygène ou un groupe NH,
B représente une liaison directe, un groupe -CH₂- (méthylène) ou -CH₂CH₂-(1,2-éthylène), et
n vaut 0 ou 1,
et des sels de tels composés.

2. Composés de formule (I) selon la revendication 1, dans lesquels R³ représente un atome d'hydrogène, R⁶ un atome d'hydrogène, B une liaison directe et n vaut 0, et R¹, R², R⁴, R⁵ et A ont la signification indiqué dans la revendication 1, et les sels de tels composés.

3. Composés de formule (I) selon la revendication 1, dans lesquels
R¹ représente un groupe alkyle en C₁₋₄ ou un groupe alkylène en C₁₋₃ portant un substituant R⁵,
R² représente un groupe alkyle en C₁₋₄,
R³ représente un groupe hydroxyméthyle, nitro, amino ou le substituant -CH₂O-COR⁷,
R⁴ représente le substituant -A-B-R⁶,
R⁵ représente un groupe furyle, thiényle, tétrahydrofuryle, phényle ou phényle portant un ou deux substituants, identiques ou différents, choisis dans le groupe formé par un atome d'halogène, un groupe alkyle en C₁₋₄ et alcoxy en C₁₋₄,
R⁶ représente un groupe thiényle, furyle, phényle ou phényle portant un ou deux substituants, identiques ou différents, choisis dans le groupe formé par un atome d'halogène, un groupe alkyl en C₁₋₄, alcoxy en C₁₋₄, nitro, -NH-CO-NH₂ (uréido), amino, (alkyle en C₁₋₄)-carbonylamino et (alcoxy en C₁₋₄)-carbonylamino,
R⁷ représente un groupe alkylc en C₁₋₄, (alcoxy en C₁₋₄)-(alkyle en C₁₋₄), (alcoxy en C₁₋₄)-carbonyl-(alkyle en C₁₋₄) ou carboxy-(alkyle en C₁₋₄),
A représente un atome d'oxygène ou un groupe NH,
B représente un groupe -CH₂- (méthylène) ou -CH₂CH₂- (1,2-éthylène) et
n vaut 0 ou 1,
et les sels de tels composés.

4. Composés de formule (I) selon la revendication 1, dans lesquels
R¹ représente un groupe isobutyle ou un groupe méthylène portant un substituant R⁵,
R² représente un groupe alkyle en C₁₋₄,
R³ représente un groupe hydroxyméthyle, nitro, amino ou le substituant -CH₂O-COR⁷,
R⁴ représente le substituant -A-B-R⁶,
R⁵ représente un groupe furyle, thiényle, phényle ou phényle portant un ou deux substituants, identiques ou différents, choisis dans le groupe formé par un atome d'halogène, un groupe alkyle en C₁₋₄ et alcoxy en C₁₋₄,
R⁶ représente un groupe thiény]e, phényle ou phényle portant un ou deux substituants, identiques ou différents, choisis dans le groupe formé par un atome d'halogène, un groupe alkylc en C₁₋₄, alcoxy en C₁₋₄, -NH-CO-NH₂ (uréido), amino, (alkyle en C₁₋₄)-carbonylamino et (alcoxy en C₁₋₄)-carbonylamino,
R⁷ représente un groupe alkyle en C₁₋₄, (alcoxy en C₁₋₄)-(alkyle en C₁₋₄), (alcoxy en C₁₋₄)-carbonyl-(alkyle en C₁₋₄) ou carboxy-(alkyle en C₁₋₄),
A représente un atome d'oxygène ou un groupe NH,
B représente un groupe -CH₂- (méthylène) et
n vaut 0 ou 1,
et les sels de tels composés.

5. Composés de formule (I) selon la revendication 1, dans lesquels
R¹ représente un groupe méthylène portant un substituant R⁵,
R² représente un groupe alkyle en C₁₋₄,
R³ représente un groupe hydroxyméthyle, amino ou le substituant -CH₂O-COR⁷,
R⁴ représente le substituant -A-B-R⁶,
R⁵ représente un groupe phényle ou phényle portant un substituant choisi dans le groupe formé par un atome de chlore ou de fluor et un groupe alcoxy en C₁₋₄,
R⁶ représente un groupe phényle ou un groupe phényle portant un substituant choisi dans le groupe formé par un atome de chlore ou de fluor,
R⁷ représente un groupe méthyle, méthoxyméthyle, méthoxycarbonylméthyle, méthoxycarbonyléthyle, carboxyméthyle ou carboxyéthyle,
A représente un atome d'oxygène,
B représente un groupe -CH₂- (méthylène) et
n vaut 0,
et les sels de tels composés.

6. 1-benzyl-7-(4-fluorobenzyloxy)-3-hydroxyméthyl-2-méthyl-pyrrolo-[2,3-d]-pyridazine et les sels de ce composé.

7. Procédé de préparation des composés de formule (I) selon la revendication 1 et de leurs sels, caractérisé par le fait que
a) l'on soumet des composés de formule (II) 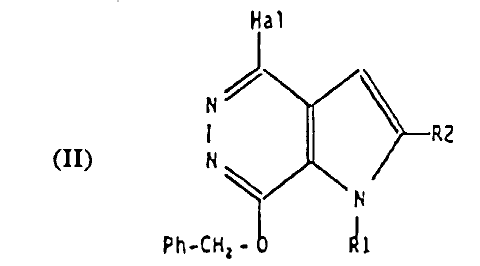 dans laquelle R¹ et R² ont la signification indiquée dans la revendication 1 et Hal représente un atome d'halogène, de préférence un atome de chlore, à une réaction de débenzylation réductrice et de déshalogénation, pour obtenir des composés de formule (I) dans lesquels R³ et R⁶ représentent un atome d'hydrogène, R⁴ représente le substituant -A-B-R⁶, A représente un atome d'oxygène, B une liaison directe et n vaut 0, ou que
b) l'on fait réagir les composés de formule (III) obtenus selon a) 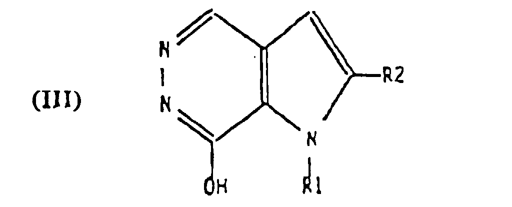 dans laquelle R¹ et R² ont la signification indiquée dans la revendication 1, avec des agents d'halogénation appropriés, pour obtenir des composés de formule (I) dans lesquels R³ représente un atome d'hydrogène, R⁴ représente un atome d'halogène et n vaut 0 ; ou que
c) l'on fait réagir les composés de formule (IV) obtenus selon b) 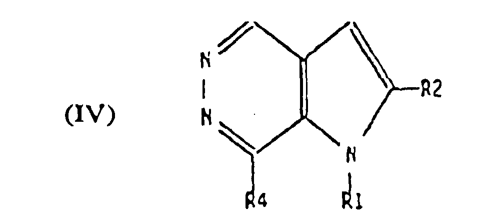 dans laquelle R⁴ représente un atome d'halogène, avec des composés de formule H-A-B-R⁶ (= composés de formule (V)), dans lesquels A, B et R⁶ ont la signification indiquée dans la revendication I, ou avec les sels d'addition de base de tels composés, pour obtenir des composés de formule (I) dans lesquels R³ représente un atome d'hydrogène, R⁴ représente le substituant -A-B-R⁶ et n vaut 0, ou que
d) l'on fait réagir des composés de formule (IV) dans lesquels R¹, R² et R⁴ ont la signification indiquée dans la revendication 1 avec des agents d'halogénation appropriés, pour obtenir des composés de formule (I) dans lesquels R³ représente un atome d'halogène et n vaut 0 ; ou que
e) l'on soumet des composés de formule (I) dans lesquels R³ représente un atome d'halogène, à une métallation intermédiaire en position 3 suivie d'une réaction avec du diméthylformamide ou avec un ester d'acide formique, pour obtenir des composés de formule (I) dans lesquels R³ représente un groupe CHO (formyle) et n vaut 0 ; ou que
f) l'on soumet des composés de formule (I) dans lesquels R³ représente un groupe CHO (formyle) à une réduction pour obtenir des composés de formule (I) dans lesquels R³ représente un groupe hydroxyméthyle et n vaut 0 ; ou que
g) l'on soumet des composés de formule (I) dans lesquels n vaut 0 à une oxydation pour obtenir des composés de formule (I) dans lesquels n vaut 1 ; ou que
h) l'on fait réagir des composés de l'armure (I) dans lesquels R⁴ représente un résidu -A-B-R⁶, où R⁶ représente un groupe phényle portant un substituant amino, avec de l'halogénoformiate d'alkyle en C₁₋₄ ou avec un halogénure d'un acide carboxylique en C₁₋₄ pour obtenir des composés de formule (I) dans lesquels R⁴ représente un résidu -A-B-R⁶ où R⁶ représente un groupe phényle portant un substituant (alcoxy en C₁₋₄)-carbonylamino ou (alkyle en C₁₋₄) carbonylamino ; ou que
i) l'on soumet des composés de formule (I) dans lesquels R³ représente un atome d'hydrogène et R⁴ un atome d'halogène, à une nitration pour obtenir des composés de formule (1) dans lesquels R³ représente un groupe nitro et R⁴ un atome d'halogène ; ou que
j) l'on fait réagir des composés de formule (I) obtenus selon i) dans lesquels R³ représente un groupe nitro et R⁴ un atome d'halogène, avec des composés de formule H-A-B-R6 (= composés de formule (V)) dans lesquels A, B et R⁶ ont la signification indiquée ci-dessus, ou avec les sels d'addition de base de tels composés, pour obtenir des composés de formule (I) dans lesquels R³ représente un groupe nitro et R⁴ le substituant -A-B-R⁶ ; ou que
k) l'on soumet des composés de formule (I) obtenus selon j) dans lesquels R³ représente un groupe nitro et R⁴ le substituant A-B-R⁶, à une réduction pour obtenir des composés de formule (I) dans lesquels R³ représente un groupe amino et R⁴ le substituant -A-B-R⁶ ; ou que
l) l'on fait réagir des composés de formule (I) dans lesquels R³ représente un groupe hydroxyméthyle, avec des composés de formule R⁷-CO-Z (= composés de formule (VI)) dans lesquels R⁷ a la signification indiquée ci-après et Z représente un groupe hydroxyle ou un groupe partant approprié, pour obtenir des composés de formule (I) dans lesquels R³ représente le substituant -CH₂O-CO-R⁷ et R⁷ représente un groupe alkyle en C₁₋₄, (alcoxy en C₁₋₄)-(alkyle en C₁₋₄), (alcoxy en C₁₋₄)-carbonyl-(alkyle en C₁₋₄) ou carboxy-(alkyle en C₁₋₄),
et que l'on convertit éventuellement si on le souhaite les composés de formule (I) obtenus selon a), b), c), d ), e), f), g), h), i), j), k), ou l) en leurs sels correspondants, ou que l'on libère éventuellement les composés de formule (I) à partir des sels des composés de formule (I) obtenus.

8. Médicament contenant un ou plusieurs composés selon ou une ou plusieurs des revendications 1 à 6 et/ou des sels pharmacologiquement acceptables de tels composés.

9. Composés selon une ou plusieurs des revendications 1 à 6 et leurs sels pharmacologiquement acceptables destinés à l'application pour la prévention et le traitement de maladies gastrointestinales.

10. Utilisation de composés selon une ou plusieurs des revendications 1 à 6 et de leurs sels pharmacologiquement acceptables pour la préparation de médicaments destinés à la prévention ou au traitement de maladies gastrointestinales.
